# EUROPEAN PATENT APPLICATION

(11) **EP 4 306 148 A1**
(43) Date of publication of application: **17.01.2024**
(21) Application number: 22184328.7
(22) Date of filing: 12.07.2022
(51) Int. Cl.: A61M 5/20, A61M 5/315, A61M 5/50, A61M 5/24

(54) **DOSE SETTING MECHANISM AND METHOD OF LOCKING AN INJECTION PEN**

(71) Applicant: medmix Switzerland AG, 9469 Haag (Rheintal) (CH)
(72) Inventor: FOCHT, Kenneth Allen, Needham, 02492 (US); COSTELLO, Peter Calvin, Raynham, 02767 (US)
(74) Representative: Manitz Finsterwald Patent- und Rechtsanwaltspartnerschaft mbB

(57) **Abstract**

A dose setting mechanism configured to set a desired dose for an injection device, the dose setting mechanism comprising a housing, a dose setting knob and a dose setting device, wherein said dose setting knob is configured to set the desired dose by a compulsory guided combined axial and rotational movement of the dose setting knob relative to the housing, wherein said dose setting mechanism comprises one or more coupling means, and wherein, on drug delivery, said dose setting knob is moved axially relative to the dose setting device and/or the housing and said one or more coupling means are configured to engage at least one of the dose setting device and the housing to thereby permanently axially lock the dose setting knob to the respective one of the dose setting device and the housing.

A Method of locking an injection pen following first use of the injection pen, the method comprising the steps of: setting a dose by a compulsory guided combined axial and rotational movement of the dose setting knob relative to the housing, and moving a dose setting knob of a dose setting mechanism axially relative to a dose setting device and/or a housing, thereby activating one or more coupling means to permanently axially lock the dose setting knob.

## Description

The invention relates to a dose setting mechanism and a method of locking an injection pen.

Injection pens are usually used for therapies which require recurring drug injections. Such therapies include insulin therapy and growth hormone therapy. An injection pen commonly comprises a dose setting mechanism that is configured to set a dose required for the therapy. Usually multiple different doses can be set depending on how much drug is needed for the individual therapy.

Some drugs need to be prepared, e.g. by mixing two or more ingredients, right before drug delivery. In order to prepare the drug, a reconstitution member can be provided with the pen. After preparing the drug, the drug should be taken as one dose in the amount required by the therapy. The remaining drug left in the pen should be discarded together with the pen.

It is an object of the invention to provide a dose setting mechanism that ensures that a dose can be reliably set and only said dose is delivered to a patient.

The object is satisfied by a dose setting mechanism according to claim 1. The dose setting mechanism is configured to set a desired dose for an injection device. The dose setting mechanism comprises a housing, a dose setting knob and a dose setting device. The dose setting knob is configured to set a dose by a compulsory guided combined axial and rotational movement of the dose setting knob relative to the housing. The dose setting mechanism comprises one or more coupling means. On drug delivery, said dose setting knob is moved axially relative to the dose setting device and/or the housing and said one or more coupling means are configured to engage at least one of the dose setting device and the housing to thereby permanently axially lock the dose setting knob to the respective one of the dose setting device and the housing.

The concept is based on the idea that it should be made sure that a user of the dose setting mechanism, e.g. a patient or a doctor, should not be able to reuse the dose setting mechanism to set a second dose after delivery of a first dose, e.g. to ensure that only freshly prepared drug is delivered to the patient.

Embodiments of the dose setting mechanism are defined by the dependent claims and described in the following disclosure.

In general, the dose setting mechanism is configured to set a desired dose for a single use injection device that is rendered inoperable when the dose setting knob is axially locked to the respective one of the dose setting device and the housing.

According to an embodiment, the dose setting knob is non-rotatable relative to the dose setting device and/or the housing when the dose setting knob is axially locked to the respective one of the dose setting device and the housing.

According to a further embodiment, said one or more coupling means are configured to cooperate between the dose setting device and the dose setting knob.

Preferably, said dose setting knob comprises said one or more coupling means configured to cooperate with the respective one of the dose setting device and the housing.

According to an embodiment, the one or more coupling means are arranged on an outer circumferential surface of the dose setting knob and corresponding coupling means, e.g. a circumferentially extending ledge, are arranged on an inner circumferential surface of the dose setting device.

In order to provide a simple but reliable coupling between the dose setting knob and the dose setting device or the housing, the one or more coupling means can comprise one or more snap elements, in particular one or more elastically deformable tongues.

The one or more snap elements can have a chamfered surface that is configured to engage with a protrusion, in particular a circumferentially extending ledge, to elastically deform the snap element.

In order to achieve the elastic deformability of the coupling means, e.g. the one or more snap elements, a cut-out can be provided next to at least one of the one or more coupling means.

The cut-out can be provided sectionally around the at least one of the one or more coupling means, e.g. the one or more snap elements.

According to an embodiment, the dose setting device comprises one or more projections for setting said desired dose and cooperating with a dose selector.

According to a further embodiment, the dose setting knob is coupled to a dose selector in a rotationally fixed manner on drug delivery. Alternatively or additionally, the dose setting knob may rotate relative to the housing while the dose selector may not rotate relative to the housing on dose setting.

Preferably, the dose setting knob forms an end face for manually pushing the dose setting knob in an axial direction relative to the dose setting device and/or the housing on drug delivery.

According to an embodiment, the dose setting knob and the dose setting device are axially moveable relative to one another on dose setting by rotating the dose setting knob relative to the housing.

According to a further embodiment, the dose setting device and the dose setting knob are rotationally fixed to each other during dose setting and/or wherein the dose setting device and the dose setting knob rotate relative to each other on drug delivery.

Preferably, the dose setting device rotates relative to the housing on drug delivery. Alternatively or additionally, the dose setting knob may not rotate relative to the housing on drug delivery.

According to an embodiment, the dose setting knob and the dose setting device are axially moveable relative to a housing on dose setting.

According to a further embodiment, the dose setting knob and the dose setting device are configured to not move relative to one another on dose setting.

Preferably, the dose setting knob comprises an injection button and said one or more coupling means are configured to permanently axially lock said injection button to the respective one of the dose setting device and the housing.

According to an embodiment, at least a part of the dose setting knob is axially moved on dose dispensing. According to an embodiment, the dose setting knob comprises an injection button and said injection button is axially moved on dose dispensing.

According to a further embodiment, on drug delivery, said dose setting knob is moved axially relative to said dose setting device and said one or more coupling means are configured to engage the dose setting device to thereby permanently axially lock the dose setting knob to the dose setting device due to the relative movement of the dose setting knob and the dose setting device.

The invention also concerns an injection device comprising a dose setting mechanism according to one or more of the above or below mentioned features.

The invention also concerns a single use and single dose pen with one or more doses being selectable for the single use with a dose setting mechanism according to one or more of the above or below mentioned features.

The invention also concerns a set comprising an injection device or a single use and single dose pen with one or more of the above or below mentioned features and a separate element, e.g. a clip, that is provided to prevent a premature moving of the dose setting knob and/or the injection button, e.g. due to the dose delivery mechanism falling onto the proximal end of said dose delivery mechanism, relative to the housing before the separate element is removed. According to an embodiment, a holding element, e.g. a knob cover, is provided that has to be removed before the separate element can be removed.

According to an embodiment, the separate element has a C-shaped cross-section. Preferably, the separate element can be attachable to the injection device or pen so that the separate element covers a region between a proximal edge of the injection button and a distal edge of the housing. Preferably, the separate element is attachable to the injection device or pen by snapping the separate element onto the injection device or pen.

The invention also concerns a method of locking an injection pen following first use of the injection pen, the method comprising the steps of: moving a dose setting knob of a dose setting mechanism axially relative to a dose setting device and/or a housing, thereby activating one or more coupling means to permanently axially lock the dose setting knob. The dose setting can be done by a compulsory guided combined axial and rotational movement of the dose setting knob relative to the housing.

The general idea is to permanently axially lock the dose setting knob to prevent a reuse of the injection pen.

According to an embodiment, the method comprises the steps of:
moving said dose setting knob of the dose setting mechanism axially relative to the dose setting device and/or the housing, thereby moving one or more coupling means of the dose setting knob into engagement with the dose setting device or the housing and axially locking the dose setting knob to the dose setting device and/or the housing.

According to a further embodiment, the method includes moving at least a part of the dose setting knob on dose dispensing.

The method can further include axially moving an injection button on dose dispensing. The injection button is preferably a part of the dose setting knob.

The moving of the dose setting knob of the dose setting mechanism axially relative to the dose setting device and/or the housing is preferably done by manually pushing the dose setting knob in an axial direction.

According to an embodiment, the dose setting knob is axially moved backwards relative to the housing to set a desired dose for an injection device prior to moving of the dose setting knob of the dose setting mechanism axially forward relative to the dose setting device and/or the housing.

According to a further embodiment, the dose setting knob is axially moved backwards relative to the housing by rotating the dose setting knob relative to the housing. This movement can be achieved by at least one thread being formed on an element of the dose setting mechanism.

Generally, the before and below mentioned features are also disclosed in combination with a dose setting mechanism configured to set a desired dose for an injection device, the dose setting mechanism comprising a housing, a dose setting knob and a dose setting device, wherein said dose setting mechanism comprises one or more coupling means, and wherein, on drug delivery, said dose setting knob is moved axially relative to the dose setting device and/or the housing and said one or more coupling means are configured to engage at least one of the dose setting device and the housing to thereby permanently axially lock the dose setting knob to the respective one of the dose setting device and the housing.

These and other features, aspects and advantages are described below with reference to the drawings. Like reference characters denote corresponding features consistently throughout the drawings.
- Figure 1: shows an exploded view of an injection pen according to the invention.
- Figure 2A: shows a perspective view of a knob cover of the injection pen of Fig. 1.
- Figure 2B: shows a side view of the knob cover of Fig. 2A.
- Figure 2C: shows a section view of the knob cover of Fig. 2B along the line A-A of Fig. 2B.
- Figure 3A: shows a perspective view of an injection button of the injection pen of Fig. 1.
- Figure 3B: shows a side view of the injection button of Fig. 3A.
- Figure 3C: shows a section view of the injection button of Fig. 3B along the line A-A of Fig. 3B.
- Figure 3D: shows a section view of the injection button of Fig. 3B along the line B-B of Fig. 3B.
- Figure 3E: shows a section view of the injection button of Fig. 3B along the line C-C of Fig. 3B.
- Figure 4A: shows a perspective view of a snap ring of the injection pen of Fig. 1.
- Figure 4B: shows a top view of the snap ring of Fig. 4A.
- Figure 4C: shows a side view of the snap ring of Fig. 4A.
- Figure 4D: shows a bottom view of the snap ring of Fig. 4A.
- Figure 5A: shows a first perspective view of a dose setting knob of the injection pen of Fig. 1.
- Figure 5B: shows a second perspective view of the dose setting knob of Fig. 5A.
- Figure 5C: shows a side view of the dose setting knob of Fig. 5A.
- Figure 5D: shows a section view of the dose setting knob of Fig. 5A along line A-A of Fig. 5C.
- Figure 6A: shows a perspective view of a snap element of the injection pen of Fig. 1.
- Figure 6B: shows a side view of the snap element of Fig. 6A.
- Figure 6C: shows a section view of the snap element of Fig. 6A along the line A-A of Fig. 6B.
- Figure 7A: shows a perspective view of a connector of the injection pen of Fig. 1.
- Figure 7B: shows a bottom view of the connector of Fig. 7A.
- Figure 7C: shows a side view of the connector of Fig. 7A.
- Figure 7D: shows a top view of the connector of Fig. 7A.
- Figure 8A: shows a first perspective view of a dose selector of the injection pen of Fig. 1.
- Figure 8B: shows a bottom view of the dose selector of Fig. 8A.
- Figure 8C: shows a side view of the dose selector of Fig. 8A.
- Figure 8D: shows a section view of the dose selector of Fig. 8A along the line A-A of Fig. 8C.
- Figure 8E: shows a section view of the dose selector of Fig. 8A along the line B-B of Fig. 8C.
- Figure 9: shows a second perspective view of the dose selector of Fig. 8A.

- Figure 10A: shows a perspective view of a knob key of the injection pen of Fig. 1.
- Figure 10B: shows a side view of the knob key of Fig. 10A.
- Figure 11A: shows a perspective view of a housing of the injection pen of Fig. 1.
- Figure 11B: shows a side view of the housing of Fig. 11A.
- Figure 11C: shows a section view of the housing of Fig. 11A along the line A-A of Fig. 11B.
- Figure 12A: shows a first side view of a dose setting sleeve of the injection pen of Fig. 1.
- Figure 12B: shows a second side view of the dose setting sleeve of Fig. 12A.
- Figure 12C: shows a third side view of the dose setting sleeve of Fig. 12A.
- Figure 12D: shows a fourth side view of the dose setting sleeve of Fig. 12A.
- Figure 12E: shows a front view of the dose setting sleeve of Fig. 12A.
- Figure 12F: shows a first perspective view of the dose setting sleeve of Fig. 12A.
- Figure 12G: shows a second perspective view of the dose setting sleeve of Fig. 12A.
- Figure 13A: shows a perspective view of a driver of the injection pen of Fig. 1.
- Figure 13B: shows a first side view of the driver of Fig. 13A.
- Figure 13C: shows a second side view of the driver of Fig. 13A.
- Figure 13D: shows a section view of the driver of Fig. 13A along the line A-A of Fig. 13C.
- Figure 14A: shows a first perspective view of a nut of the injection pen of Fig. 1.
- Figure 14B: shows a second perspective view of the nut of Fig. 14A.
- Figure 14C: shows a side view of the nut of Fig. 14A.
- Figure 14D: shows a first section view of the nut of Fig. 14A along the line A-A of Fig. 14C.
- Figure 14E: shows a second section view of the nut of Fig. 14A along the line A-A of Fig. 14C.
- Figure 15A: shows a first side view of a piston rod guide of the injection pen of Fig. 1.
- Figure 15B: shows a second side view of the piston rod guide of Fig. 15A.
- Figure 15C: shows a section view of the piston rod guide of Fig. 15A along the line A-A of Fig. 15A.
- Figure 15D: shows a perspective view of the piston rod guide of Fig. 15A.
- Figure 16A: shows a first longitudinal section view of the piston rod guide of Fig. 15A.
- Figure 16B: shows a second longitudinal section view of the piston rod guide of Fig. 15A.
- Figure 16C: shows a perspective view of the piston rod guide of Fig. 15A.
- Figure 17A: shows a side view of a piston rod of the injection pen of Fig. 1.
- Figure 17B: shows a section view of the piston rod of Fig. 17A along the line A-A of Fig. 17A.
- Figure 17C: shows a first perspective view of the piston rod of Fig. 17A.
- Figure 17D: shows a second perspective view of the piston rod of Fig. 17A.
- Figure 18A: shows a perspective view of a piston disc of the injection pen of Fig. 1.
- Figure 18B: shows a top view of the piston disc of Fig. 18A.
- Figure 18C: shows a section view of the piston disc of Fig. 18A along the line A-A of Fig. 18B.
- Figure 19A: shows a perspective view of a dual chamber cartridge of the injection pen of Fig. 1.
- Figure 19B: shows a side view of the dual chamber cartridge of Fig. 19A.
- Figure 19C: shows a section view of the dual chamber cartridge of Fig. 19A along the line A-A of Fig. 19B.
- Figure 20A: shows a perspective view of a cartridge container of the injection pen of Fig. 1.
- Figure 20B: shows a first side view of the cartridge container of Fig. 20A.
- Figure 20C: shows a second side view of the cartridge container of Fig. 20A.
- Figure 20D: shows a section view of the cartridge container of Fig. 20A along the line A-A of Fig. 20C.
- Figure 21A: shows a first perspective view of a cartridge key of the injection pen of Fig. 1.
- Figure 21B: shows a second perspective view of the cartridge key of Fig. 21A.
- Figure 21C: shows a first side view of the cartridge key of Fig. 21A.
- Figure 21D: shows a second side view of the cartridge key of Fig. 21A.
- Figure 22A: shows a third side view of the cartridge key of Fig. 21A.
- Figure 22B: shows a section view of the cartridge key of Fig. 21A along the line A-A of Fig. 22A.
- Figure 23A: shows a side view of the injection pen of Fig. 1 in an as-delivered state.
- Figure 23B: shows a section view of the injection pen of Fig. 23A along the line A-A of Fig. 23A.
- Figure 24: shows a perspective view of the injection pen of Fig. 23A without the knob cover and with some parts displayed transparently.
- Figure 25A: shows a second side view of the injection pen of Fig. 23A.
- Figure 25B: shows a section view of the injection pen of Fig. 23A along the line A-A of Fig. 25A.
- Figure 26A: shows a side view of the injection pen of Fig. 1 in a reconstitution state.
- Figure 26B: shows a section view of the injection pen of Fig. 26A along the line A-A of Fig. 26A.
- Figure 27A: shows a second side view of the injection pen of Fig. 26A.
- Figure 27B: shows a third side view of the injection pen of Fig. 26A.
- Figure 27C: shows a section view of the injection pen of Fig. 26A along the line A-A of Fig. 27B.
- Figure 28A: shows a side view of the injection pen of Fig. 1 in a knob cover unfastening state.
- Figure 28B: shows a section view of the injection pen of Fig. 28A along the line A-A of Fig. 28A.
- Figure 29A: shows a side view of the injection pen of Fig. 1 in an end of reconstitution state.
- Figure 29B: shows a section view of the injection pen of Fig. 29A along the line A-A of Fig. 29A.
- Figure 30A: shows a side view of the injection pen of Fig. 1 in a set dose state.
- Figure 30B: shows a section view of the injection pen of Fig. 30A along the line A-A of Fig. 30A.
- Figure 31A: shows a side view of the injection pen of Fig. 1 in a start of injection state.
- Figure 31B: shows a section view of the injection pen of Fig. 31A along the line A-A of Fig. 31A.
- Figure 32A: shows a further side view of the injection pen of Fig. 1 in a start of injection state.
- Figure 32B: shows an enlarged section view of the injection pen of Fig. 32A along the line A-A of Fig. 32A.
- Figure 33A: shows a side view of the injection pen of Fig. 1 in an end of injection state.
- Figure 33B: shows a section view of the injection pen of Fig. 33A along the line A-A of Fig. 33A.
With reference to figures 1 to 22B, parts of an injection pen 10 according to the invention are described. Afterwards, with reference to figures 23A to 33B it is described how the pen is meant to be used.

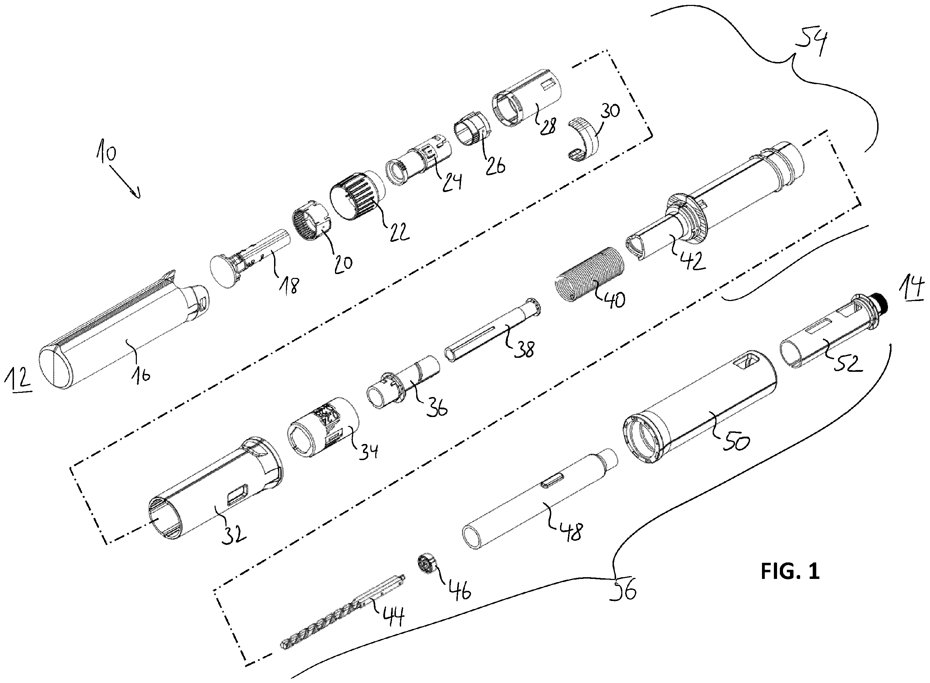

Fig. 1 shows an exploded view of a medicament delivery device in form of an injection pen 10. The injection pen 10 comprises - in an order from a distal end 12 to a proximal end 14 - a knob cover 16 that can also be called knob lock, cover or holding element, an injection button 18 that can also be called clutch and can be part of an actuation member, a snap ring 20, a dose setting knob 22 that can also be called dose adjusting member or knob and can be part of an actuation member, a snap element 24 that can also be called dose setting device, a connector 26, a dose selector 28, a knob key 30 that can also be called a clip, a housing 32 that can also be called body, a dose setting sleeve 34 that can also be called dose sleeve or dose indication member, a driver 36, a nut 38, a spring 40, a piston rod guide 42 that can also be called piston guide, a piston rod 44, a piston disc 46 that can also be called plunger, a dual chamber cartridge 48 that can also be called fluid compartment or cartridge, a cartridge container 50 , and a cartridge holder or cartridge key 52.

The different parts can be grouped together to define different functional units. E.g. the section between the injection button 18 and the piston rod guide 42 can be called a dose setting mechanism 54, a dose setting unit, a dose delivery mechanism and/or a dose delivery activation mechanism. On the other hand, the section between the piston rod guide 42 and the cartridge key 52 can be called drug reconstitution unit 56 or reconstitution means.

Next, the above-mentioned parts of the injection pen 10 are described in the order starting from the distal end 12 and ending at the proximal end 14:

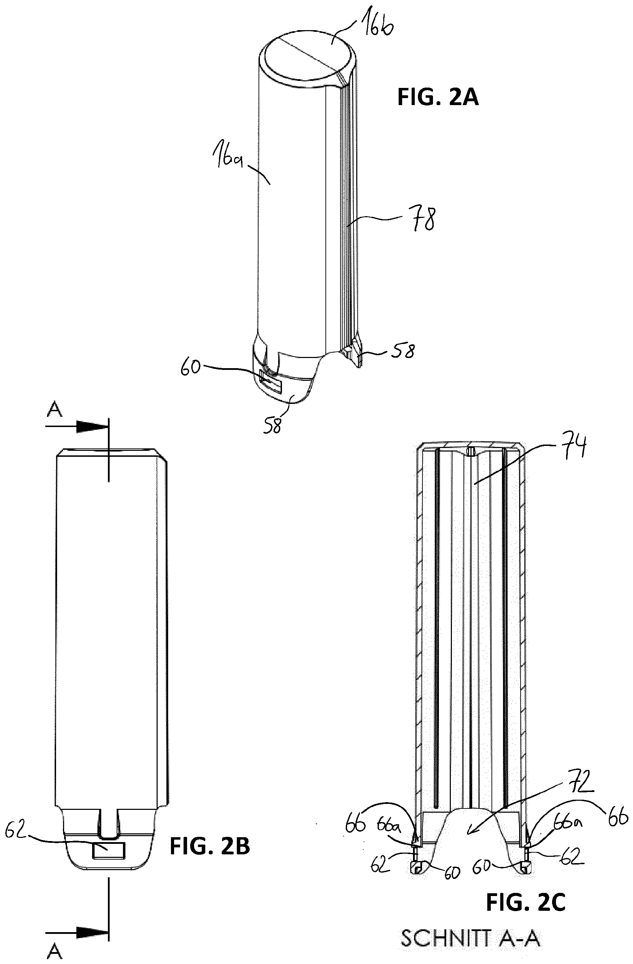

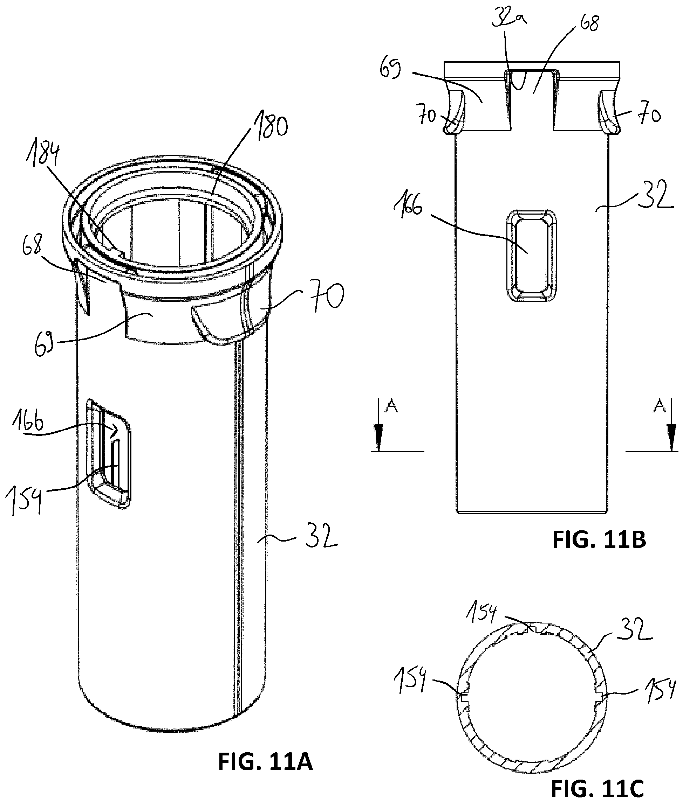

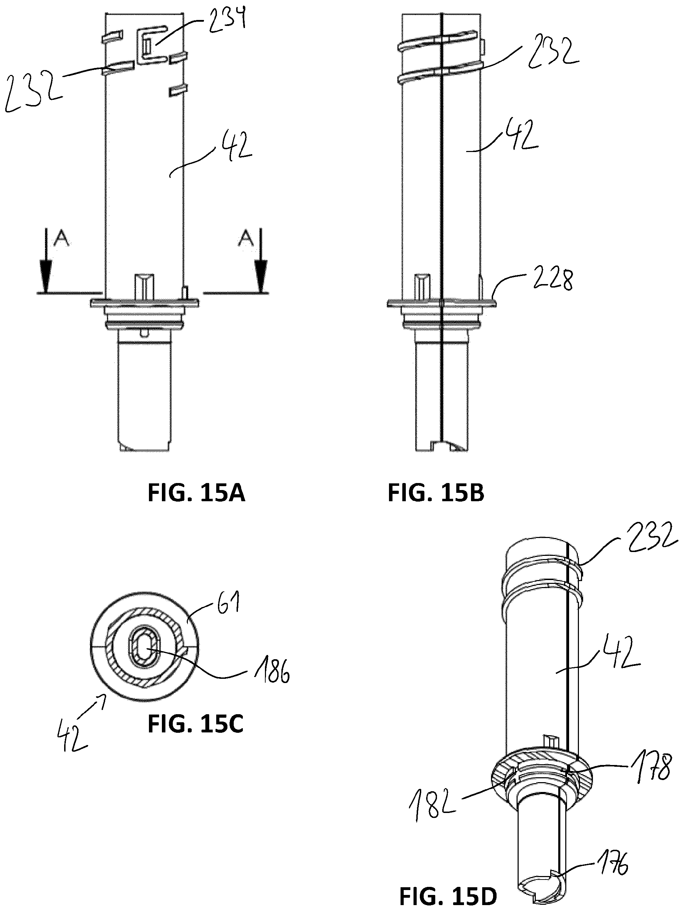

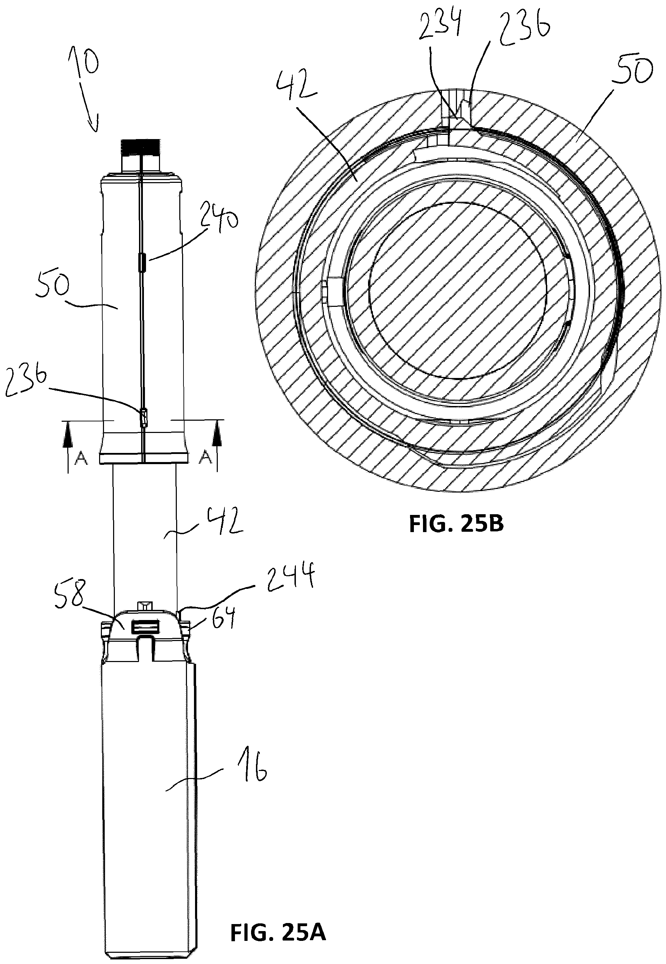

Figs. 2A to 2C depict the knob cover 16. The knob cover 16 covers the dose setting knob 22 during delivery, i.e. shipping, of the injection pen 10 to a costumer, e.g. the patient. The knob cover 16 is fully detachable from the rest of the injection pen 10. The knob cover 16 is attachable to the housing 32 and/or detachable from the housing 32 via two deformable wings 58 that can be deflected outwardly, i.e. in a radial direction, to detach the knob cover 16 from the housing 32. The wings 58 form a proximal end section of the knob cover 16. On an inner surface of each of the wings 58, form-fitting engagement means in the form of a lug 60 are provided, that are configured to engage with the housing 32, in particular with a radially extending coupling surface 228 (cf. Fig. 15C) formed on the piston guide 42, to axially fixate the knob cover 16 relative to the housing 32 in a distal direction. Next to each of the lugs 60, one window 62, i.e. a radially extending opening, is formed in the wings 58. When the knob cover 16 is attached to the housing 32, the windows 62 are positioned at an axial position where the housing 32 forms a circumferentially extending elevation 64 (cf. Fig. 25A). On the distal side of each window 62, i.e. away from the lugs 60, on the inner side surface of the respective wing 58, an abutment 66 is formed. The abutment 66 has a width that is adapted to a width of a recess or cut-out 68 (cf. Fig. 11A) on an outer surface of the housing 32, more precisely in a chamfered portion 69 formed on the outer surface of the housing 32. Furthermore, the abutment 66 forms a front surface 66a that axially abuts a radially extending surface 32a (cf. Fig 11B) defining a proximal end of the cut-out 68 when the knob cover 16 is attached to the housing 32. The radially extending surface 32a defines a stop surface that stops proximal movement of the knob cover 16 relatively to the housing 32, e.g. if the injection pen 10 is dropped onto a floor with the distal end 12 first. In order to further ensure that the knob cover 16 does not move past its attached position in the proximal direction, axial abutment elevations 70 (cf. Fig. 11A) can be formed on the outer surface of the housing 32. The elevations 70 are configured to engage with clearances 72 (cf. Fig. 2C) formed between the wings 58 so that proximal front surfaces of the knob cover 16 abut distal front faces of the axial abutment elevations 70.

A form-fitting engagement between the abutments 66 and the cut-outs 68 and/or a form-fitting engagement between the elevations 70 and the clearances 72 make sure that the knob cover 16 is rotationally constrained relative to the housing 32 when the knob cover 16 is attached to the housing 32.

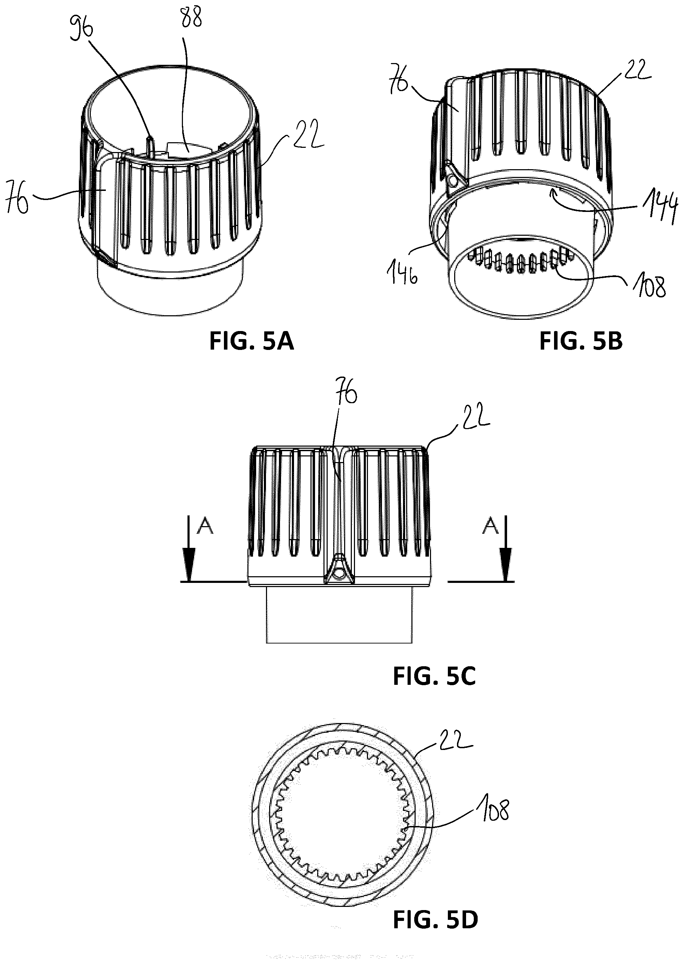

As can be seen from Fig. 2A, the knob cover 16 is only detachable from the rest of the injection pen 10 by moving the knob cover 16 linearly in a distal direction. In order to do so, a linear recess 74 is formed on the inner circumferential surface of the knob cover 16 that corresponds to an anti-rolling means 76 (cf. Fig. 5B) of the dose setting knob 22 in the form of an axially extending rib. Therefore, the dose setting knob 22 is blocked from rotating inside the knob cover 16 by the form-fitting engagement of the linear recess 74 and the anti-rolling means 76. The knob cover 16, as can be seen in Fig. 2A, also forms anti-rolling means 78 in form of an axially extending rib on the outer surface of the knob cover 16. The anti-rolling means 76 and 78 make sure that the injection pen 10 and the knob cover 16 do not roll away when placed on a flat surface. As can be also seen from Fig. 2A, the knob cover 16 has a closed circumference 16a and a closed face 16b at its distal end. Therefore, the knob cover 16 forms a closed sleeve around the distal section of the injection pen 10.

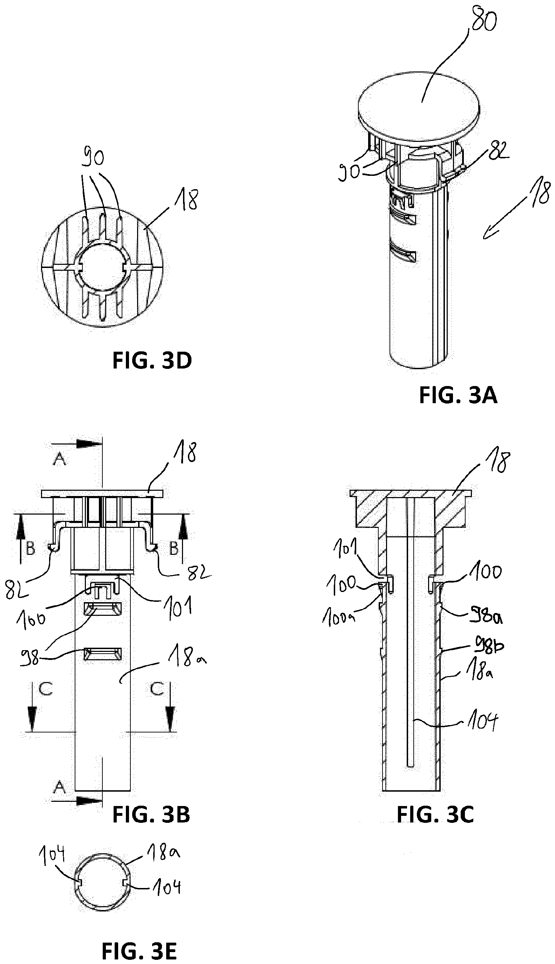

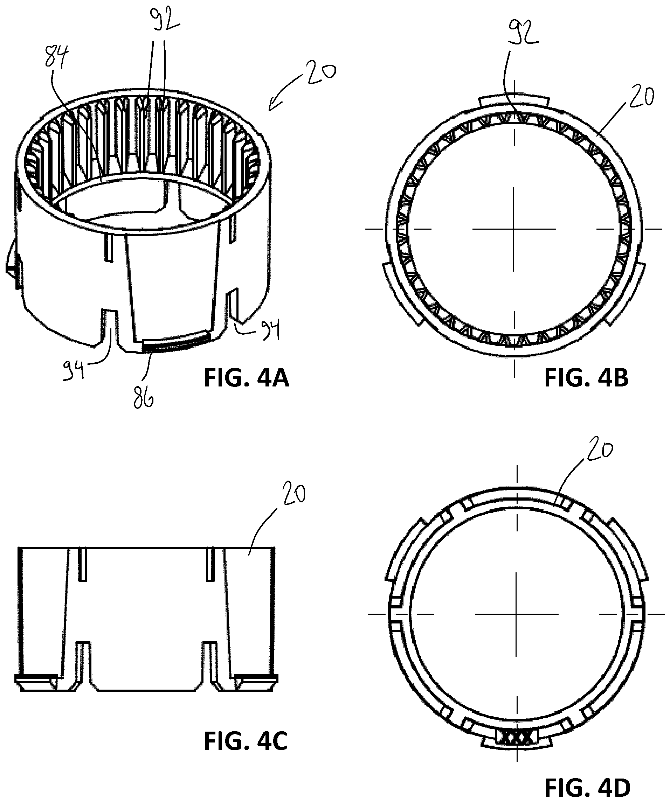

Figures 3A to 3E depict the injection button 18. The injection button 18 forms a distal front surface 80 to apply a force to the injection button 18 to inject a set dose. The injection button 18 comprises axial fixation means 82 to axially attach the injection button 18 to the snap ring 20 (cf. Fig. 4A-4D) which is axially connected to the dose setting knob 22 (cf. Fig. 5A to 5D). The axial fixation means 82 comprise two elastically deformable hooks 82 which engage with a circumferentially extending rib 84 on the snap ring 20. The snap ring 20 also comprises axial fixation means 86 in the form of elastically deformable bendable hooks that engage with an undercut 88 formed in the dose setting knob 22. The injection button 18, the snap ring 20 and the dose setting knob 22 are permanently axially fixed to each other.

The injection button 18 also forms rotation fixation means 90 in the form of radially extending ribs. The ribs 90 are form-fittingly engaged with rotation fixation means 92 (cf. Fig. 4A) in the form of teeth arranged in an inner circumferential surface of the snap ring 20 to rotationally connect the injection button 18 to the snap ring 20. The snap ring 20 comprises rotation fixation means 94 in the form of axially extending recesses that define side surfaces of the elastically deformable bendable hooks 86 and that engage with rotation fixation means 96 in the form of axially extending ribs (cf. Fig. 5A) on the inner circumferential surface of the dose setting knob 22.

After assembly, the injection button 18, the snap ring 20 and the dose setting knob 22 are rigidly connected with each other and form both a dose setting member and an actuation member of the dose delivery mechanism 54.

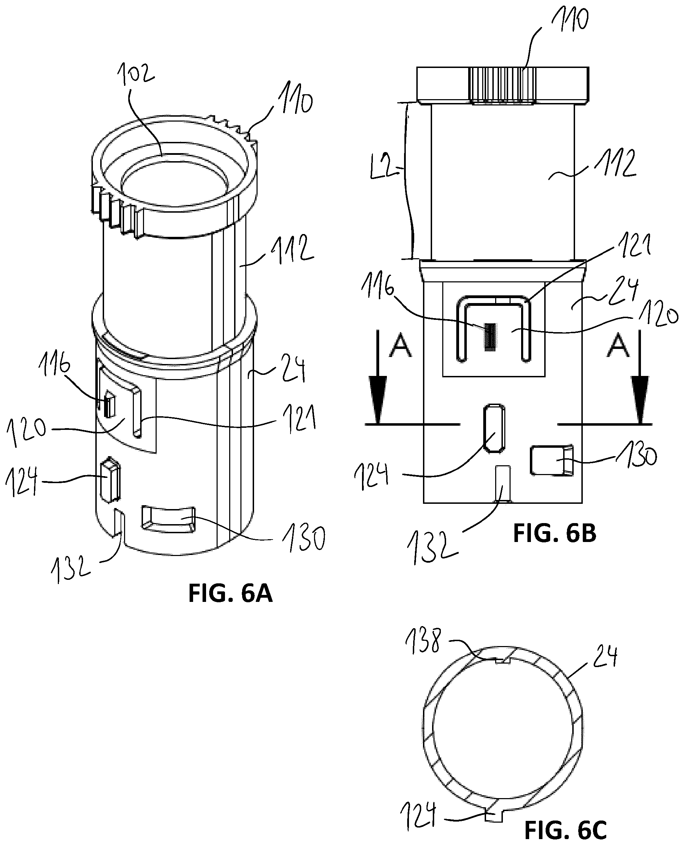

The injection button 18 forms a cylindrical portion 18a. On the cylindrical portion 18a, assembling means 98 in the form of elevations are formed to axially preassemble the injection button 18 with the snap element 24. More precisely, the lower, i.e. proximal, assembling means 98b (cf. Fig. 3C) restricts distal movement of the injection button 18 relative to the snap element 24 by interfering with coupling means 102 on the snap element 24. The upper, i.e. distal, assembling means 98a restricts proximal movement of the injection button 18 relative to the snap element 24 by interfering with coupling means 102 on the snap element 24 after pre-assembly and distal movement of the injection button 18 after final assembly. When the snap element 24 and the injection button 18 are preassembled, i.e. in a preassembled state, the coupling means 102 is arranged between the proximal assembling means 98b and the distal assembling means 98a. In said preassembled state, the injection button 18 is not yet rigidly connected to the snap ring 20 and the dose setting knob 22. However, when the coupling means 102 is arranged distally from the distal assembling means 98a, i.e. in the assembled state, the injection button 18 is rigidly connected to the snap ring 20 and the dose setting knob 22. The injection button 18 also forms coupling means 100 in the form of protrusions being arranged on an outer circumferential surface of the injection button 18 on elastically inwardly bendable portions. The inwardly bendable portions extend in an axial direction and are sectionally surrounded by cut-outs 101. The coupling means 100 are configured to permanently axially lock the injection button 18 and therefore also the snap ring 20 and the dose setting knob 22 to the snap element 24 after the injection has been completed to render the injection pen 10 inoperable. Namely, when the injection button 18 is moved axially to initiate the dose delivery, the coupling means 100 pass the radially inwardly extending coupling means in the form of a circumferentially extending ledge 102 (cf. Fig. 6A) on the snap element 24. The radially inwardly extending ledge 102 causes the protrusions being arranged on elastically inwardly bendable portions 100 to bend inwardly until the protrusions have passed the ledge 102. In order to reduce the force needed to push the protrusions 100 past the ledge 102, the protrusions 100 form chamfered outer surfaces 100a. Alternatively or additionally, the ledge 102 could form a chamfered inner surface. When the protrusions 100 have passed the ledge 102, they snap back into their neutral position which causes the injection button 18 to be permanently axially locked relative to the snap element 24. This feature makes sure that the injection pen 10 can only be used one single time to inject exactly one dose.

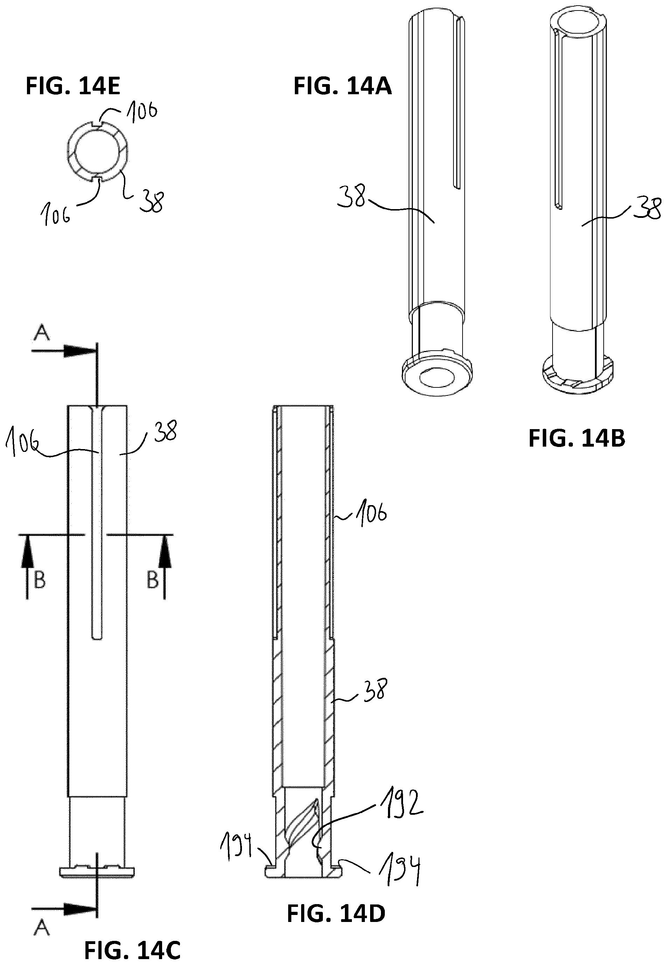

As can be best seen in Fig. 3C and 3E, the injection button 18 comprises an axially extending rib 104 on its inner circumferential surface. The axially extending rib 104 engages in an axially extending groove 106 of the nut 38 (cf. Fig. 14C) to form rotation fixation means. Due to the axially extending rib 104 and the corresponding axially extending groove 106, the injection button 18 and the nut 38 can move axially relative to each other but are rotationally fixed to each other.

As can be best seen on Fig. 5B and 5D, a set of teeth 108 are formed in an axial section on an inner circumferential side of the dose setting knob 22. These teeth 108 are configured to mesh with a set of teeth 110 arranged in a distal section on an outer circumferential surface of the snap element 24 (cf. Fig. 6A-6C) during dose setting. Therefore, when the dose setting knob 22 is rotated during dose setting, the snap element 24 is rotated too.

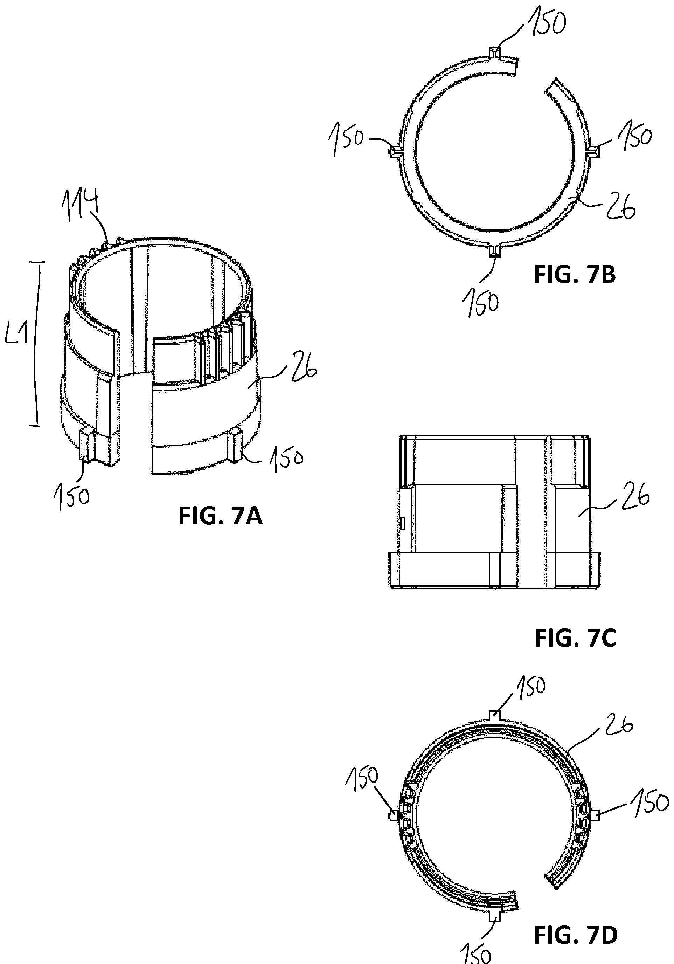

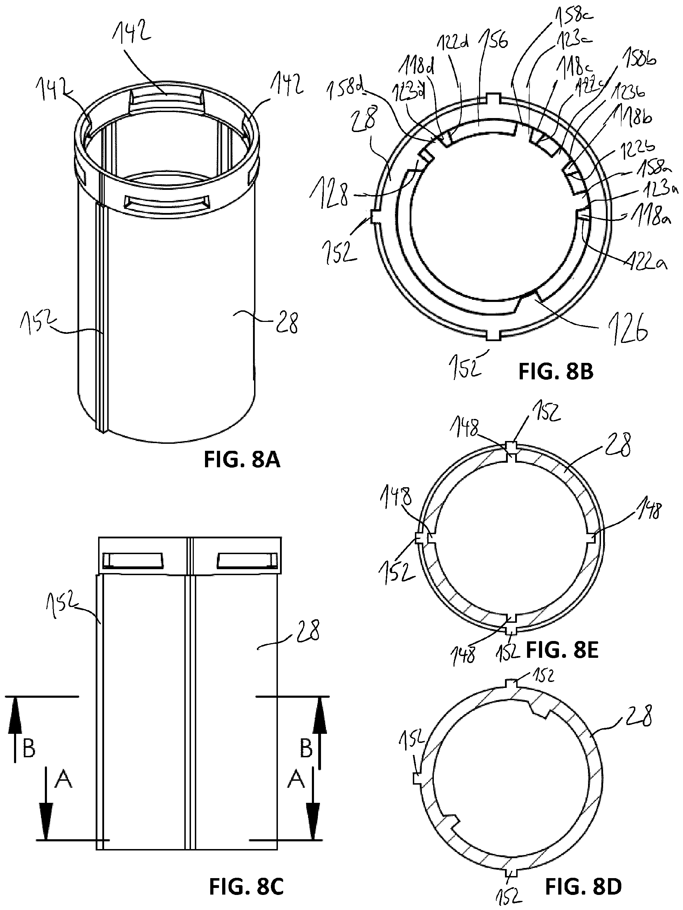

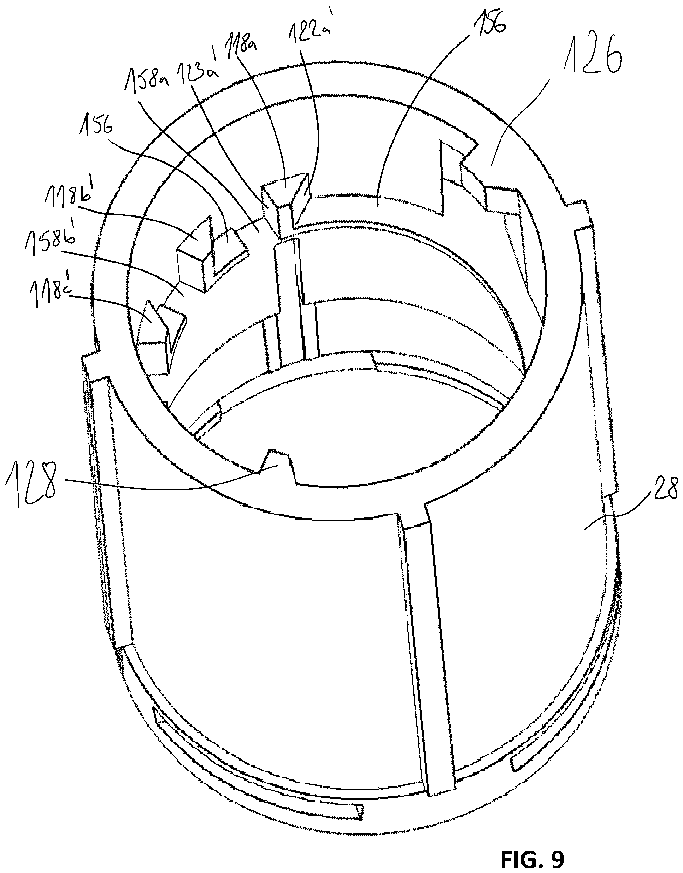

The snap element 24 forms an axial section with a reduced cross section forming a coupling surface 112 for the connector 26. The connector 26 has an open cross section (cf. Fig. 7D) so it is clippable onto the snap element 24 at the reduced cross section. The connector 26 is axially fixedly connected to the snap element 24 in both directions due to the connector 26 having a length L1 in the axial direction that corresponds to a length L2 in the axial direction of the axial section with the reduced cross section. However, the connector 26 is rotatable relative to the snap element 24. When the dose setting knob 22 is pushed in the proximal direction to initiate dose delivery, the teeth 108 of the dose setting knob 22 engage with a set of teeth 114 formed on an outer circumferential surface of the connector 106 instead of the teeth 110 of the snap element 24 so that the snap element 24 can rotate relative to the dose setting knob 22 during dose delivery. The engagement between the teeth 108 of the dose setting knob 22 and the teeth 114 of the connector 106 makes sure that the dose setting knob 22 does not rotate during dose delivery with respect to the housing 32 due to connector 106 being rotationally fixed to the housing 32 via the dose selector 28. The snap element 24 further comprises an engagement feature 116 in the form of an axially extending radial projection. The engagement feature 116 is an axially extending rib. The engagement feature 116 can have a symmetrical cross section in a radial plane perpendicular to a longitudinal axis of the injection pen 10 or an asymmetrical cross section. The engagement feature 116 is configured to engage with dose stops 118a, 118b, 118c, and 118d (cf. Fig. 8B) formed on an inner circumferential surface of the dose selector 28 to set a desired dose. Therefore, the engagement feature 116 is used as a dose definition element. The dose definition element 116 is located on an elastically deformable section 120, i.e. an axially extending arm partially surrounded by a cut-out 121. The elastically deformable section 120 bends inwardly when the dose definition element 116 passes one of the dose stops 118a, 118b, 118c, and 118d. In order to reduce the force needed to rotate the dose setting knob 22 and the snap element 24 relative to the dose selector 28 to enlarge or decrease the set dose, the dose stops 118 have chamfered side surfaces 122a-d and 123a-d. According to the embodiment shown in Fig. 8B, the dose stops 118a-d have a symmetrical cross section in the radial plane perpendicular to the longitudinal axis of the injection pen 10. In other words, the chamfered side surfaces 122 and 123 have pitches that are equal to each other regarding their amount. According to another embodiment shown in Fig. 9, chamfered side surfaces 122a'-d' that get in contact with the projection 116 to deform the elastically deformable section 120 when the dose is set to a higher dose have a smaller pitch than chamfered side surfaces 123a'-d' that get in contact with the projection 116 when the dose is set to a lower dose. The side surfaces 123a-d define rotational positions corresponding to settable doses. The spring 40 is configured to rotate the snap element 24 relative to the dose selector 28 so that the dose definition element 116 abuts one of the side surfaces 123a-d.

The snap element 24 further comprises a hard stop 124 in the form of an axially extending rib that abuts a hard stop 126 formed on the dose selector 28 when the injection pen 10 is delivered to a costumer. The hard stop 126, contrary to known pens, does not correspond to a zero-dose stop but instead corresponds to a pre-set dose stop. A further discussion regarding this feature follows. The hard stop 124 is axially distanced from the dose definition element 116 but axially aligned with the dose definition element 116. The hard stop 124 is configured to abut an end of dose setting hard stop 128.

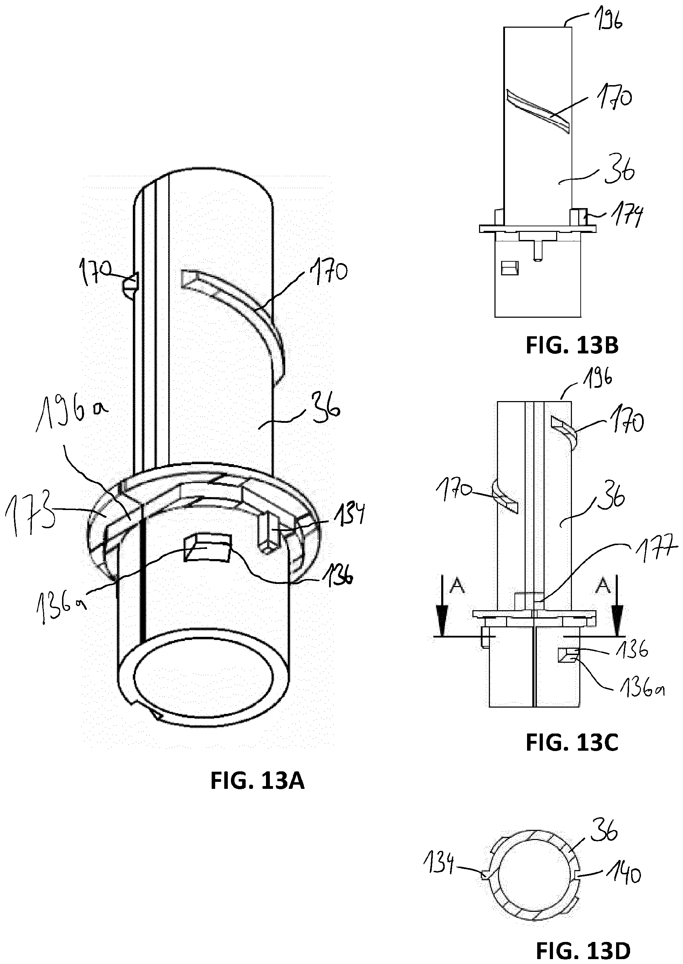

The snap element 24 further comprises axial and rotational fixation means in the form of a radially extending opening 130 and an axially extending slot 132 to axially and rotationally fix the snap element 24 to the driver 36. As can be seen in Fig. 13A, the driver 36 has an axially extending rib 134 that is configured to engage with the slot 132 of the snap element 24. Furthermore, the driver 36 has a protrusion 136 with a chamfered surface 136a that engages with the opening 130 of the snap element 24. While the opening 130 and the protrusion 136 form the axial fixation means, the slot 132 and the rib 134 form the rotational fixation means. Due to the axial and rotational fixation means, the snap element 24 and the driver 36 can be connected to each other in one defined relative rotational position. In order to strengthen the rotational fixation between the snap element 24 and the driver 36, an axially extending rib 138 is formed on an inner circumferential surface of the snap element 24 (cf. Fig. 6C) that engages with an axially extending groove 140 (cf. Fig. 13D) on an outer circumferential surface of the driver 36.

Figures 8A to 8E depict the dose selector 28. The dose selector 28 comprises axial fixation means 142 in the form of circumferentially extending projections on an inner circumferential surface of a distal section of the dose selector 28. The dose selector 28 is axially fixed to the dose setting knob 22 by inserting the distal section with the axial fixation means 142 into a circumferentially extending intake 144 (cf. Fig. 5B). In the intake 144, the dose setting knob 22 forms axial fixation means 146 in the form of circumferentially extending protrusions on an outer circumferential surface which get engaged with the axial fixation means 142 of the dose selector 28 to form an axial connection that allows relative rotational movement between the dose selector 28 and the dose setting knob 22.

As can be seen best on Fig. 8E, rotation fixation means 148 in the form of axially extending grooves are formed on an inner circumferential surface of the dose selector 28. The rotation fixation means 148 are engaged with rotation fixation means 150 in the form of axially extending ribs formed on the outer circumferential surface of the connector 26 (cf. Fig. 7B). The rotation fixation means 148, 150 allow axial movement between the dose selector 28 and the connector 26. The dose selector 28 further comprises rotation fixation means 152 in the form of axially extending ribs formed on an outer circumferential surface of the dose selector 26. The rotation fixation means 152 engage with rotation fixation means 154 in the form of axially extending grooves formed on the inner circumferential surface of the housing 32 (cf. Fig. 11C). The rotation fixation means 152, 154 are configured to define one single possible rotational alignment that allows insertion of the dose selector 28 into the housing 32. The rotation fixation means 150, 152 allow axial movement between dose selector 28 and the housing 32.

In order to define deliverable doses, the dose selector 28 (cf. Fig. 8B) forms a circumferentially extending rib 156 with cut-outs 158a, 158b, 158c, and 158d. The cut-outs 158a, 158b, 158c, and 158d are assigned to the respective dose stops 118a, 118b, 118c, and 118d. The rib 156 with its cut-outs 158a, 158b, 158c, and 158d makes sure, that injection is only possible if the dose definition element 116 of the snap element 24 is at an angular position relating to one of the cut-outs 158a, 158b, 158c, and 158d, i.e. relating to one of the settable doses. If the dose definition element 116 is not at an angular position relating to one of the cut-outs 158a-d, axial movement of the dose definition element 116, and therefore the snap element 24, relative to the dose selector 28 is blocked by the circumferentially extending rib 156. As can be seen from Fig. 8B, there is no cut-out assigned to the pre-set dose hard stop 126. Therefore, starting an injection is inhibited when the injection pen 10 is set to the pre-set dose.

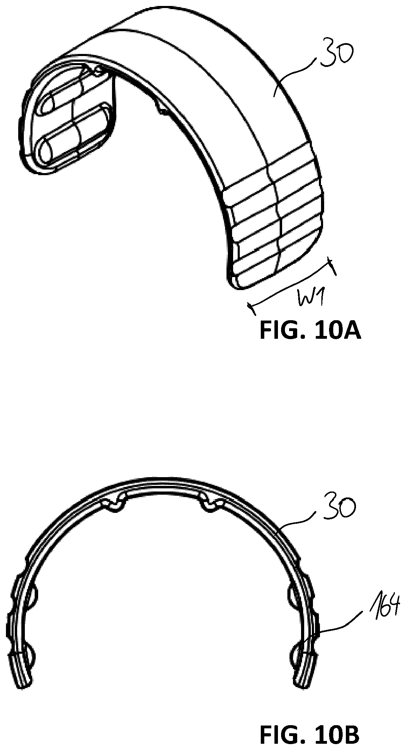

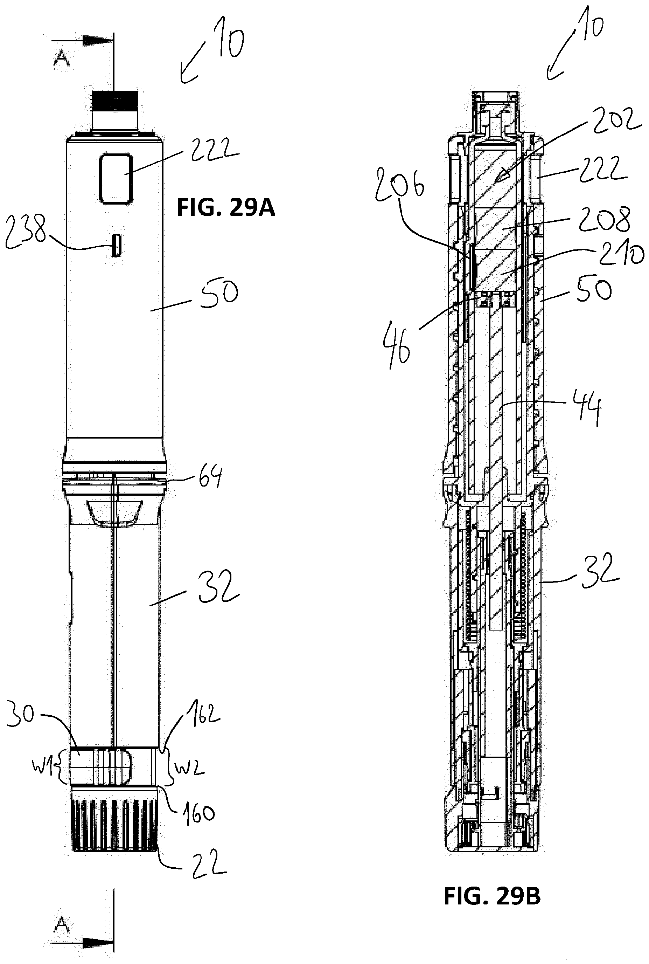

Figs. 10A and 10B depict the knob key 30. The knob key 30 is configured to be attached to the outer circumferential surface of the dose selector 28 to keep the dose setting knob 24 from unintentionally moving in the proximal direction relative to the housing 32 if the injection pen 10 in an as-delivered state drops onto its proximal end. The clip element 30 has a width W1 that corresponds to a width W2 (cf. Fig. 29A) between a proximal edge 160 of the dose setting knob 22 and a distal edge 162 of the housing 32. The knob key 30 is C-shaped and has holding protrusions 164 that interact with the rotation fixation means 152 on the outer circumferential surface of the dose selector 28 to attach the knob key 30 to the dose selector 28. The knob key 30 can be taken off the dose selector 28 by slightly bending the C-shaped knob key 30. In the as-delivered state, the knob cover 16 extends around the knob key 30 to hold the knob key 30 in place. The knob key 30 can only be taken off the dose selector 28 after the knob cover 16 has be removed.

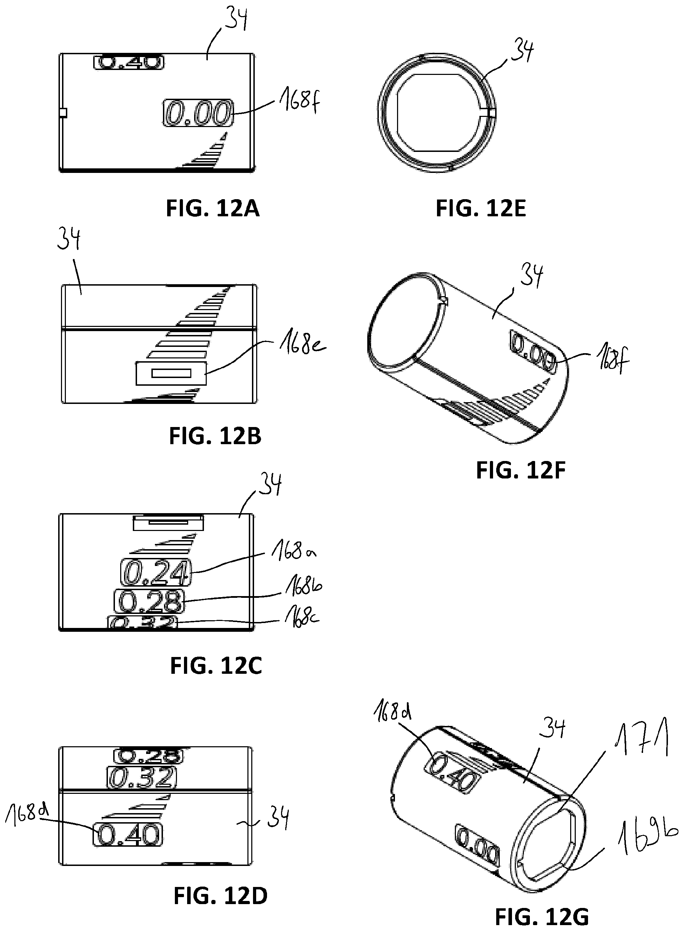

The housing 32 is shown in Figs. 11A to 11C. The housing 32 forms a viewing window 166 for displaying a state of the injection pen 10, in particular a set dose, indicated by the dose sleeve 34 through the window 166. The dose sleeve 34 rotates relative to the housing during dose setting and dose delivery which causes a change of what is displayed through the window 166. In different circumferential positions along the outer circumferential surface of the dose sleeve 34, labels 168a-168d (cf. Figs. 12C and 12D) for different settable doses are located. Furthermore, a preset-dose label 168e (cf. Fig. 12B) is located on the dose sleeve 34 that corresponds to a pre-set dose, i.e. an amount of medicament that would be injected if the injection could be started from the pre-set dose. As can be seen from comparing Figs. 12A and 12B, the pre-set dose label 168e differs from a zero-dose label 168f, i.e. the label that shows that no medicament would be injected if the injection would be started in that state. This zero-dose label 168f is shown through the window 166 when the injection has been completed. The labels 168a, 168b, 168c, and 168d correspond to the settable doses defined by the dose stops 118a, 118b, 118c, and 118d.

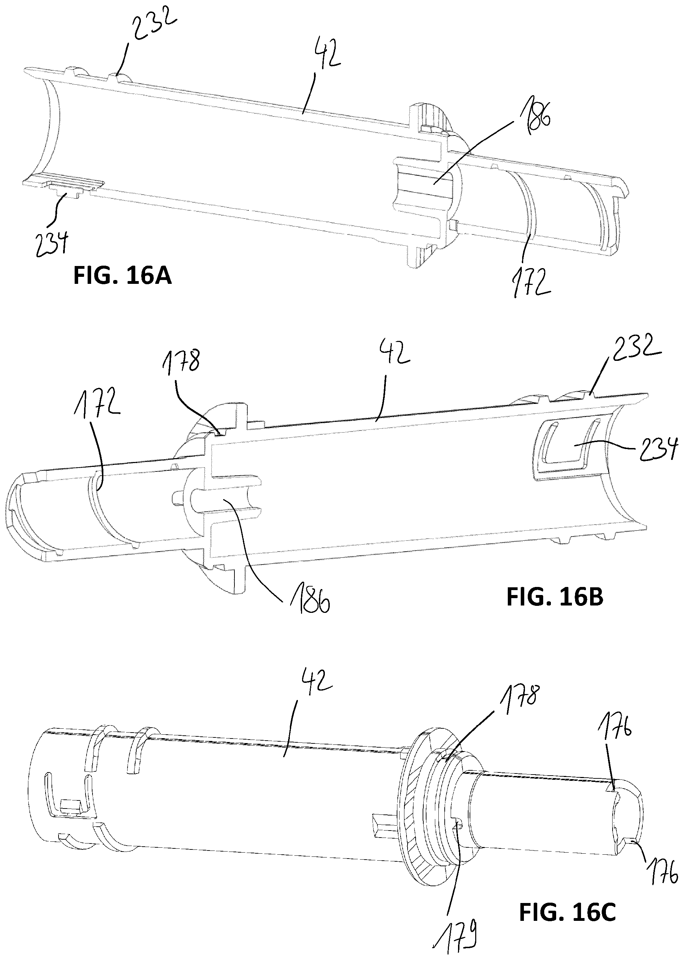

The dose sleeve 34 is rotationally and axially rigidly coupled to the driver 36 (cf. Fig 13A-13D). In order to rotationally couple the dose sleeve 34 to the driver 36 corresponding out-of-round outer and inner circumferential surfaces 169a and 169b are formed on the dose sleeve 34 and the driver 36. Furthermore, the dose sleeve 34 forms a fixing section 171 that is pinched between a proximal end of the snap element 24 and a face surface 173 (cf. Fig. 13A) of the driver 36 to axially fix the dose sleeve 34 to the driver 36 and the snap element 24. The driver 36 forms an outer thread 170 that engages with an inner thread 172 (cf. Figs. 16A-16B) of the piston guide 42. The threaded connection 170, 172 causes the driver 36 to rotate when the driver 36 is moved axially relative to the piston guide 42 and causes the driver 36 to move axially relative to the piston guide 42 when the driver 36 is rotated relative to the piston guide 42. Furthermore, the driver 36 defines end stops 174 that abut end stops 176 of the piston guide 42 at the end of the dose delivery. The surfaces defining the end stops 174, 176 are arranged in parallel to a middle axis of the injection pen and face in a radial direction. The driver 36 also forms attachment means 177 in the form of a radially extending hook for attaching one end section of the spring 40 to the driver 36. The other end section of the spring 40 is attached to attachment means 179 (cf. Fig. 16C) at the outer circumferential surface of the piston rod guide 42.

The piston guide 42 is axially and radially fixed to the housing 32 and can therefore be considered part of the housing. In order to axially fix the piston guide 42 to the housing 32, axial fixation means 178 in the form of a circumferentially extending groove are formed on the piston guide 42 that engage with axial fixation means 180 (cf. Fig. 11A) in the form of a circumferentially extending rib formed on an inner circumferential surface of the housing 32. In order to rotationally fix the piston guide 42 to the housing 32, rotation fixation means 182 in the form of an axially extending groove are formed on an outer circumferential surface of the piston guide 42 that engage with rotation fixation means 184 (cf. Fig. 11A) in the form of an axially extending rib formed on an inner circumferential surface of the housing 32. The axial and rotational fixation means 178, 180, 182, and 184 allow attachment of the piston rod guide 42 to the housing 32 in exactly one relative rotational position.

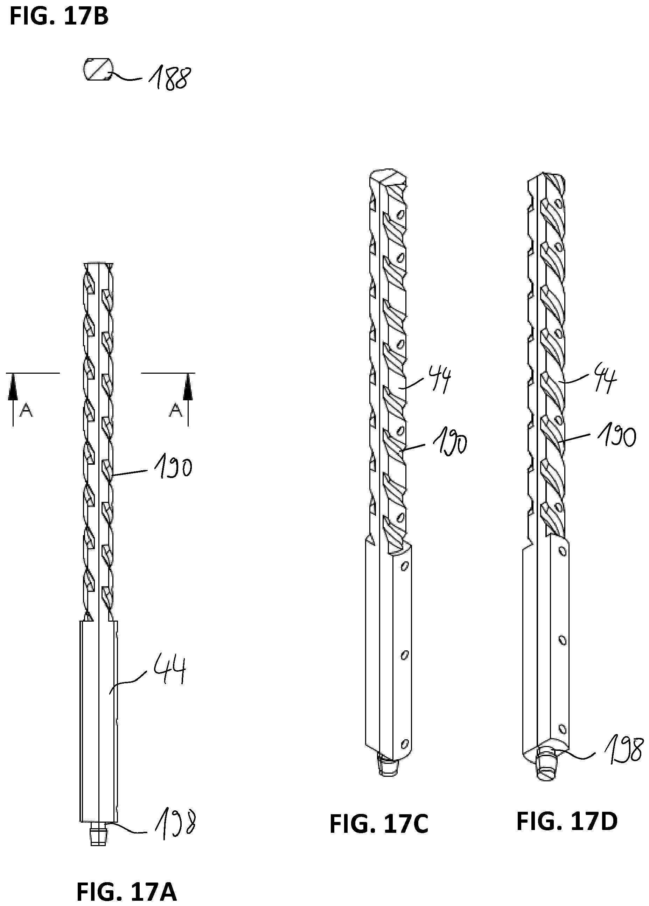

The piston guide 42 has an out of round axial opening 186 (cf. Fig. 15C) that corresponds to an out of round cross-section 188 (cf. Fig. 17B) of the piston rod 44. Therefore, the piston rod 44 is axially movable relative to the piston rod guide 42, but cannot rotate relative to the piston rod guide 42. The piston rod 44 forms an outer thread 190 that is in engagement with an inner thread 192 (cf. Fig. 14D) of the nut 38. The piston rod 44 and the nut 38 can move relative to each other in a compulsory guided combined axial and rotational movement. In a proximal end section of the nut 38, an annular pressing surface 194 extending in the distal direction is formed on the nut 38. This pressing surface 194 abuts a front surface 196 of the driver 36 during dose delivery. During dose delivery, the driver 36 moves in a combined axial and rotational movement relative to the piston rod guide 42 while the nut 38 is rotationally fixed to the housing 32. In order to reduce friction during dose delivery, a ball bearing and / or a glide disc made of low-friction material can be arranged between the pressing surface 194 and the front surface 196 of the driver 36. In both cases, during dose delivery, the driver 36 pushes the piston rod 44 via the nut 38 in the proximal direction.

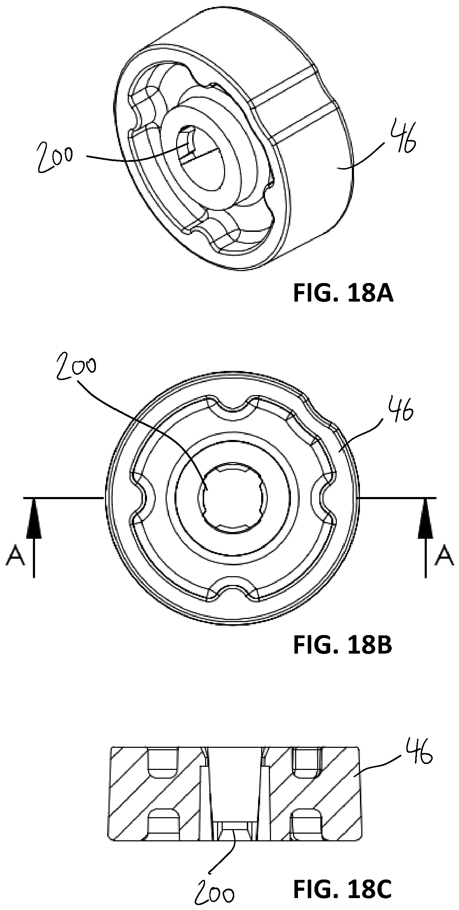

The piston rod 44, at its proximal end, forms coupling means 198 in the form of an undercut that engage with coupling means 200 in the form of radially inwardly extending ribs on an inner circumferential surface of the piston disc 46 (cf. Fig. 18A-18C).

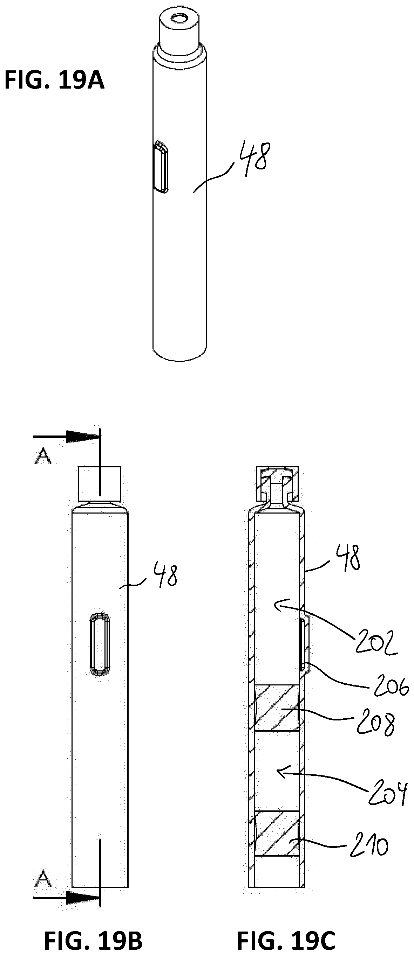

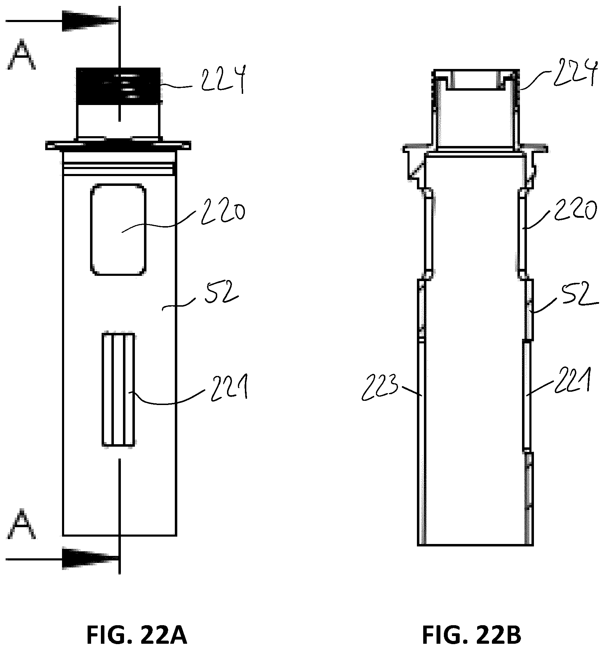

Figs. 15A to 22B depict parts of a drug mixing or reconstitution unit 56 configured to mix different components, usually a lyophilized drug and a liquid solvent, to form an injectable liquid drug. In Figs. 19A to 19C, the dual chamber cartridge 48 is shown. The dual chamber cartridge 48 is made of a transparent material such as glass. As can be seen from Fig. 19C, the cartridge 48 forms a first chamber 202 and a second chamber 204. In the as-delivered state shown in Fig. 19C, the first chamber 202 being arranged in proximal to the second chamber 204 comprises a bypass 206. The first chamber 202 and the second chamber 204 are separated by a first sealing element 208, e.g. made of a rubber material, that is axially slid ably arranged inside the dual chamber cartridge 48. In other words, the first sealing element 208 forms a distal end of the first chamber 202 and a proximal end of the second chamber 204. A second sealing element 210, e.g. made of a rubber material, forms a distal end of the second chamber 204. The piston disc 46 abuts the distal end face of the second sealing element 210 during mixture of the two components.

In the as-delivered state the lyophilized drug is in the first chamber 202 and the solvent in the second chamber 204.

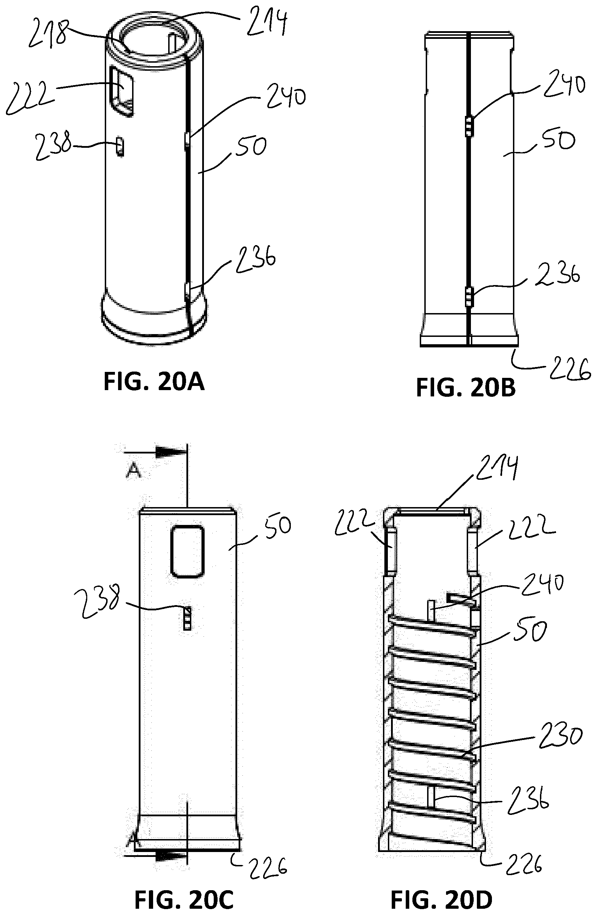

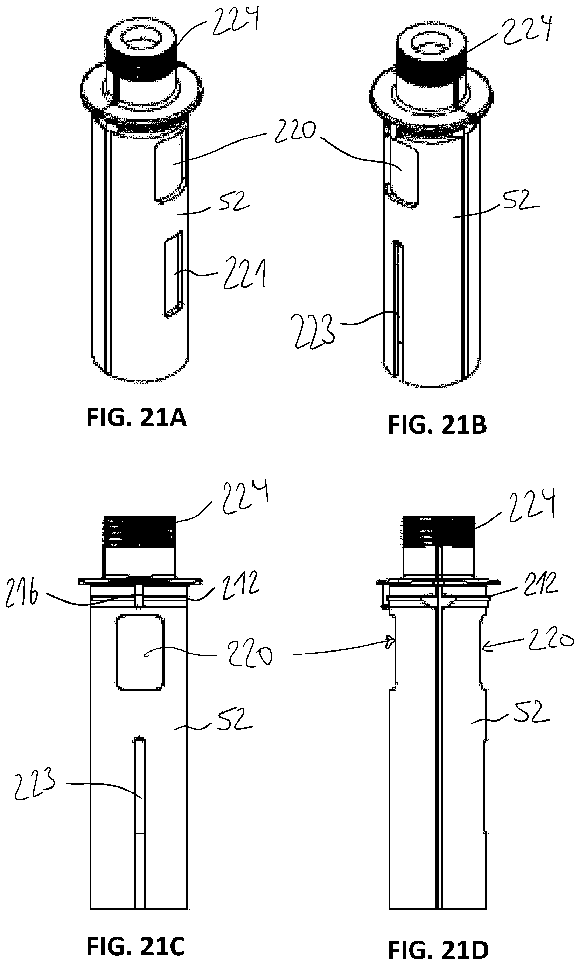

The dual chamber cartridge 48 is stored in the cartridge key 52 (cf. Fig. 21A-22b). The cartridge key 52 is axially and rotationally fixed to the cartridge container 50. To achieve that, the cartridge key 52 forms axial fixations means 212 in the form of a circumferentially extending groove that engage with axial fixation means 214 (cf. Fig. 20A) in the form of a circumferentially extending rib on an inner circumferential surface of the cartridge container 50. Furthermore, the cartridge key 52 forms rotation fixations means 216 in the form of a radially extending rib that engage rotation fixations means 218 in the form of a radially extending groove on the inner circumferential surface of the cartridge container 50. When the cartridge key 52 is attached to the cartridge container 50, a window 220 formed in the cartridge key 52 is aligned with a window 222 in the cartridge container 50 so that the patient can see the drug inside the transparent dual chamber cartridge 48 during reconstitution. At the proximal end of the cartridge key 52, which also defines the proximal end of the injection pen 10, a thread 224 is formed for attaching a needle (not shown).

The cartridge key 52 defines a cylindrical receptacle that receives the cartridge 48 and prevents tilting of the cartridge 48 with respect to the longitudinal axis. Furthermore, the cartridge key 52 forms a cut-out 221 to receive the bypass 206 of the dual chamber cartridge 48. The bypass 206 form-fittingly engages the cut-out 221 so that the dual chamber cartridge 48 is axially and rotationally fixed to the cartridge key 52. On the opposite side of the cut-out 221, a slot 223 is formed extending in the axial direction. The slot 223 allows to reversibly widen the cartridge key 52 to axially insert the dual chamber cartridge 48 with the bypass 206.

In order to mix the different components in the dual chamber cartridge 48, the cartridge container 50 is screwed onto the piston rod guide 42 until a distal end surface 226 of the cartridge container 50 abuts a proximal surface 228 (cf. Fig. 15B) of the piston guide 42. In order to screw the cartridge container 50 onto the piston rod guide 42, a first thread 230 is formed on an inner circumferential surface of the cartridge container 50 that is engaged with a second thread 232 formed on an outer circumferential surface of the piston rod guide 42. As can be seen in Fig. 15A, the piston rod guide 42 forms a snap element 234. The snap element 234 allows screwing, i.e. a compulsory guided combined axial and rotational movement, of the cartridge container 50 relative to the piston rod guide 42 in the distal direction but blocks screwing of the cartridge container 50 relative to the piston rod guide 42 in the proximal direction if the snap element 234 engages with one of the openings 236, 238, and 240. The first opening 236 (cf. Fig. 20B) is configured to define a starting position of the cartridge container 50 and makes sure that the cartridge container 50 cannot be detached from the piston rod guide 42. This starting position or as-delivered state is shown in Figs. 25A and 25B.

The second opening 238 defines a reconstitution state of the cartridge container 50. In this state, the second chamber 202 still contains air so that the injection pen 10 can be moved forth and back to ensure that the drug is homogenously mixed together. The second opening 238 may be omitted. Therefore, the present disclosure is also directed at an embodiment of the injection pen 10 that features the first 236 and third opening 240 but not the second opening 238. The third opening 240 defines a knob cover unfastening state of the cartridge container 50 where the most of the air is expelled from the second chamber 202, which now contains the reconstituted medicament ready for use.

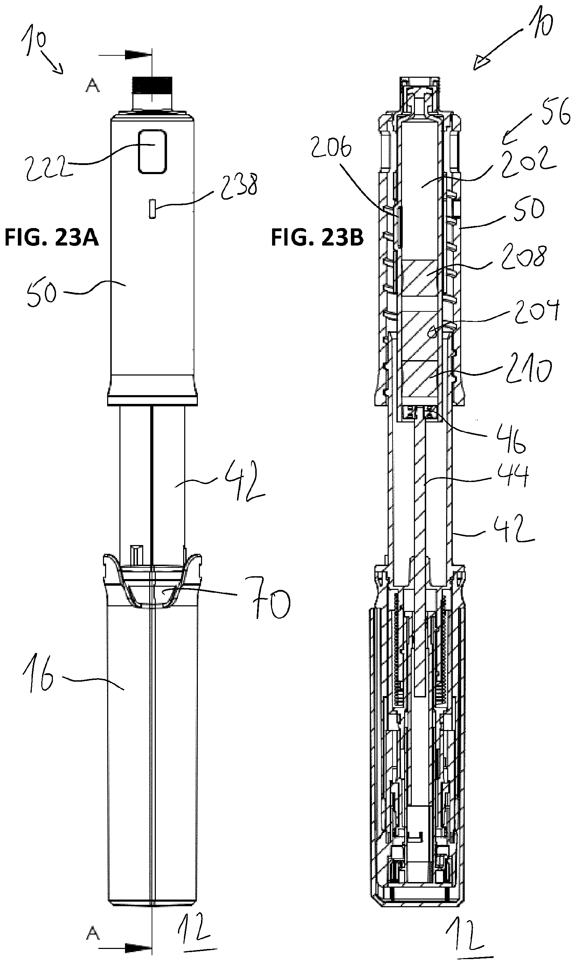

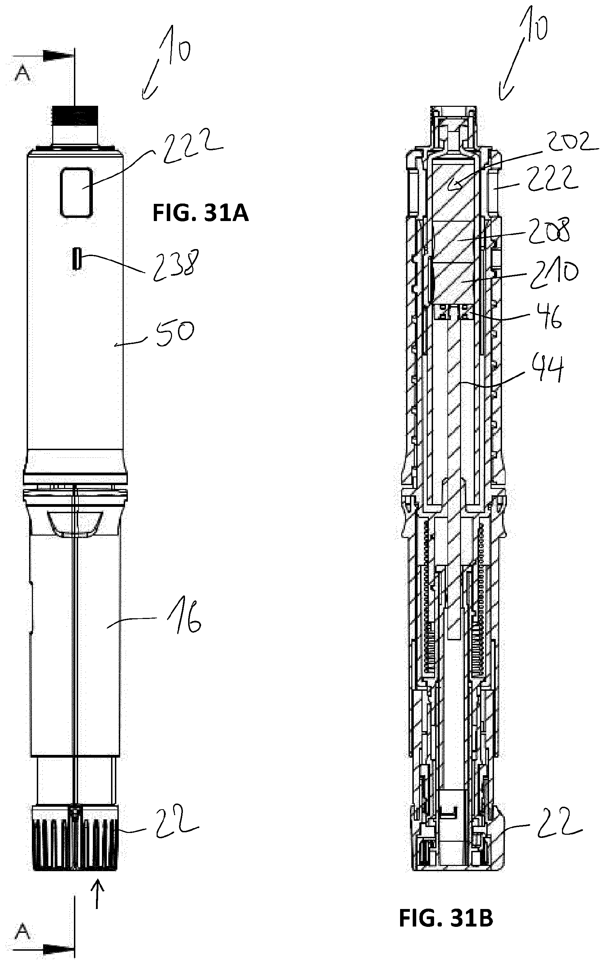

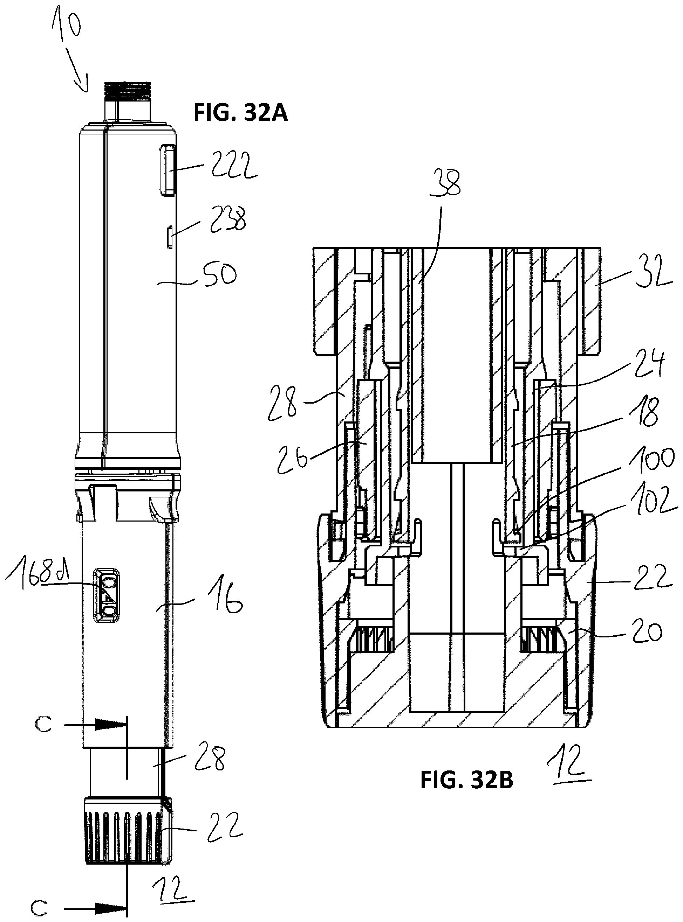

In the following with regard to Figs. 23A to 33B, different states of the injection pen 10 are described during usage of the pen 10.

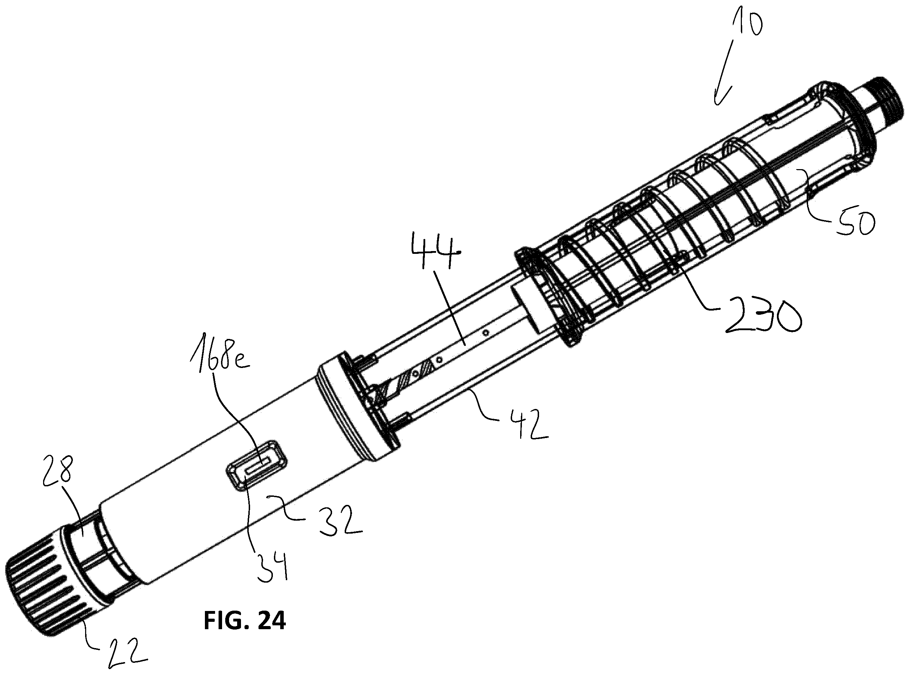

Figs. 23A to 25B depict the injection pen 10 in the as-delivered state. As can be seen in Fig. 23A, the knob cover 16 covers a distal end section of the injection pen 10 up to a joint between the housing 32 and the piston guide 42. Therefore, the dose setting knob 22 is fully covered by the knob cover 16 so that it is not possible for the user to prematurely set a dose in this state. Looking at Fig. 23B, it can be seen that in the as-delivered state, the drug reconstitution unit 56 forms two separate chambers 202, 204 divided by the first sealing element 208. That means that the two components of the drug, each being stored in one of the two chambers 202, 204 are not yet mixed together. As can be seen in Fig. 24, where the knob cover 16 is blanked out to show what is under the knob cover 16, the dose setting sleeve 34 indicates that the injection pen 10 is in a preset state which differs from a zero-dose state. Accordingly, the dose setting knob 22 is also in a preset position differing from a zero-dose position. As can be seen in Fig. 25A and 25B, the snap element 234 of the piston rod guide 42 is snapped into the first opening 236 of the cartridge container 50. In Fig. 24, the cartridge container 50 is depicted as semi-transparent in order to show the first thread 230 formed on the inner circumferential surface of the cartridge container 50. Secondly, the piston rod guide 42 is also depicted as semi-transparent to show the position of the piston rod 44 in the preset state.

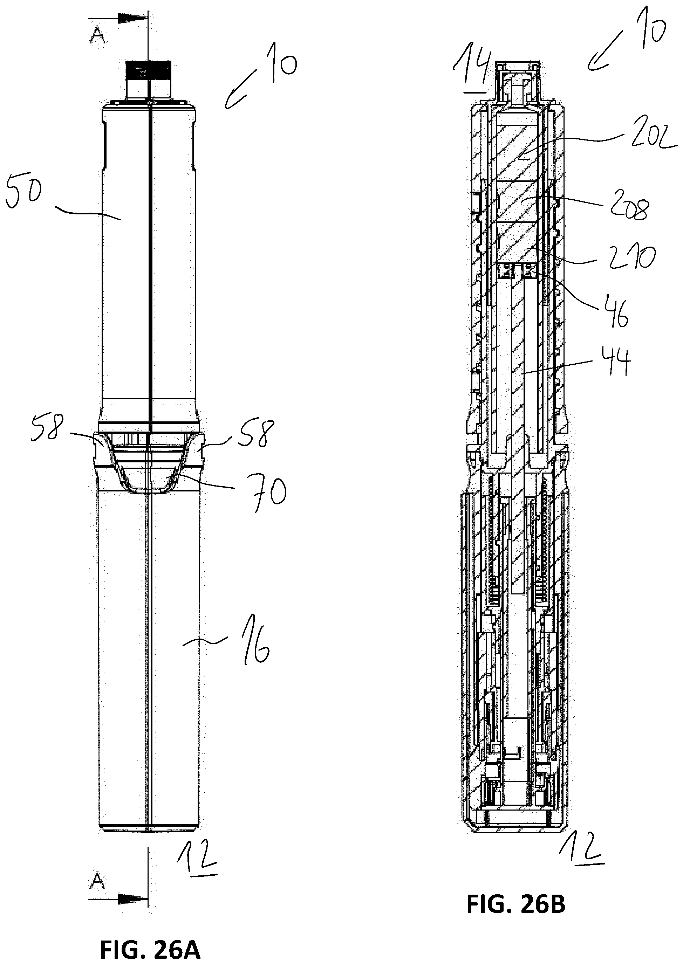

To start preparation of the drug, as can be seen from comparing Figs. 25A and 26A, the cartridge container 50 is rotated by the user which causes the cartridge container 50 including the cartridge key 52 and the dual chamber cartridge 48 to move in the distal direction relative to the piston rod guide 42. The piston disc 46 is snapped to the piston rod 44, which is rotationally fixed by the piston rod guide 42 and axially fixed by the nut 38. The piston disc 46 thus blocks the movement of the second sealing element 210 arranged in the dual chamber cartridge 48 so that the second sealing element 210 slides along the inner circumferential surface of the dual chamber cartridge 48 while the cartridge container 50 is further screwed onto the piston rod guide 42. The solvent stored in the second chamber 204 pushes against the first sealing element 208 which also causes the first sealing element 208 to slide along the inner circumferential surface of the dual chamber cartridge 48. This would cause an overpressure in the cartridge, but the air can escape through the double-ended needle the user attached to thread 224. When the first sealing element 208 reaches the bypass 206 (cf. Fig. 23B), the first chamber 202 and the second chamber 204 are connected by the bypass 206 and therefore, the lyophilized drug stored in the first chamber 202 and the solvent stored in the second chamber 204 mix.

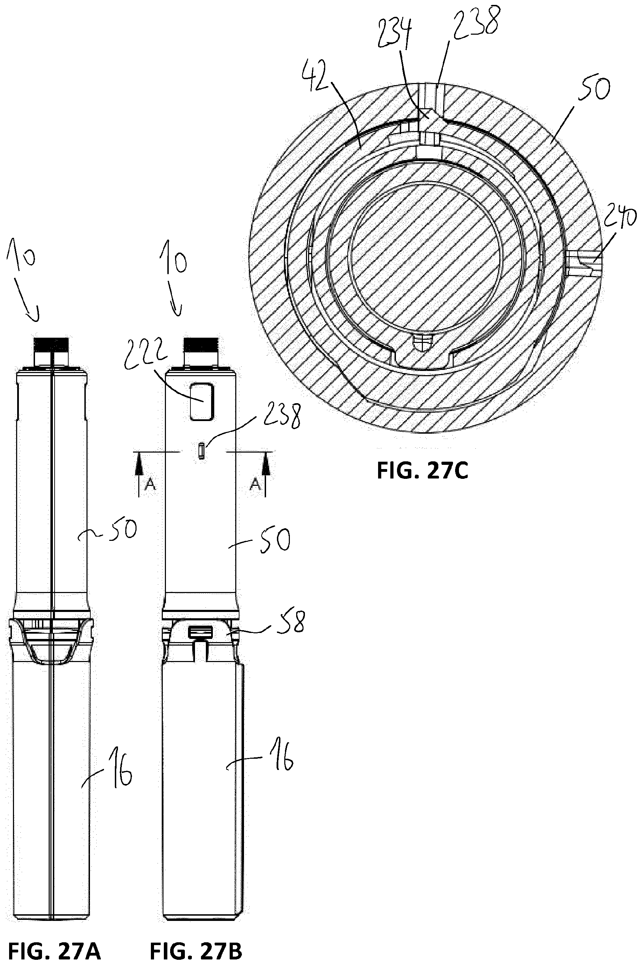

In the reconstitution state shown in Figs. 26A to 27C, the mixed drug is stored in the first chamber 202 between the first sealing element 208 and the proximal end 14 of the dual chamber cartridge 48. As can be seen in Fig. 26B, a proximal end surface of the second sealing element 210 abuts a distal end surface of the first sealing element 208 so that no second chamber 204 is present anymore in the reconstitution state. As can be seen in Fig. 27C, the snap element 234 of the piston rod guide 42 is snapped into the second opening 238 of the cartridge container 50. In this state, the front chamber 202 still contains a significant amount of air, which helps to create turbulence when moving the pen, so that the mixing of the lyophilized drug is easier. As mentioned before, the second opening 238 can be omitted. In that case the mixing takes place with a low residual amount of air.

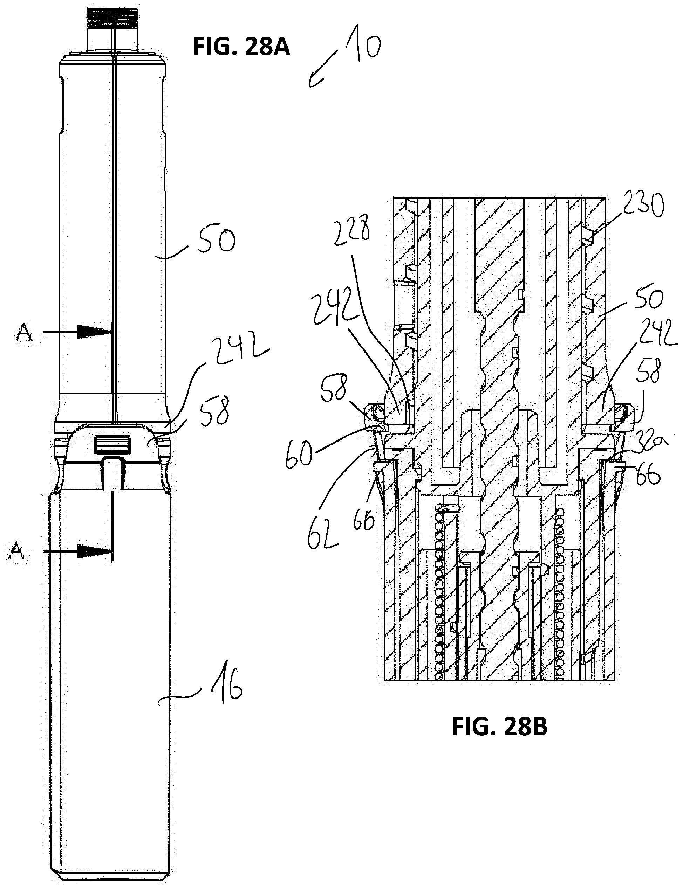

After the reconstitution of the drug is finished, the cartridge container 50 is further rotated by the user causing the cartridge container 50 to move further axially in the distal direction relative to the piston rod guide 42. This causes a displacement section 242 positioned at a distal end of the cartridge container 50 to engage with and spread the wings 58 of the knob cover 16 radially outwardly (cf. Fig. 28B). Spreading the wings 58 radially outwardly causes the form-fitting engagement means 60 of the knob cover 16 to disengage from the coupling surface 228 so that the knob cover 16 is axially movable relative to the housing 32. It is now possible to pull off the knob cover 16 from the housing 32 in the distal direction resulting in the state shown on Fig. 29A and 29B. When the cartridge container 50 is fully screwed onto the piston rod guide 42, a radial end stop 244 formed on an outer circumferential surface of the piston rod guide 42 abuts a radial end stop (not shown) on an inner circumferential surface of the cartridge container 50. Furthermore, the snap element 234 of the piston rod guide 42 is snapped into the third opening 240. Consequently, the cartridge container 50 is rotationally locked to the piston rod guide 42 and the housing 32 of the device. Therefore, movement of the cartridge container 50 and the cartridge 48 respect to the housing 32 and the piston rod guide 42 is inhibited.

As can be seen in Fig. 29A and 29B, at this stage the knob key 30 is still clipped onto the outer circumferential surface of the dose selector 28 between the proximal edge 160 of the dose setting knob 22 and the distal edge 162 of the housing 32. The knob key 30 can be taken away from the dose selector 28 only after the knob cover 16 has been removed by slightly bending the knob key 30.

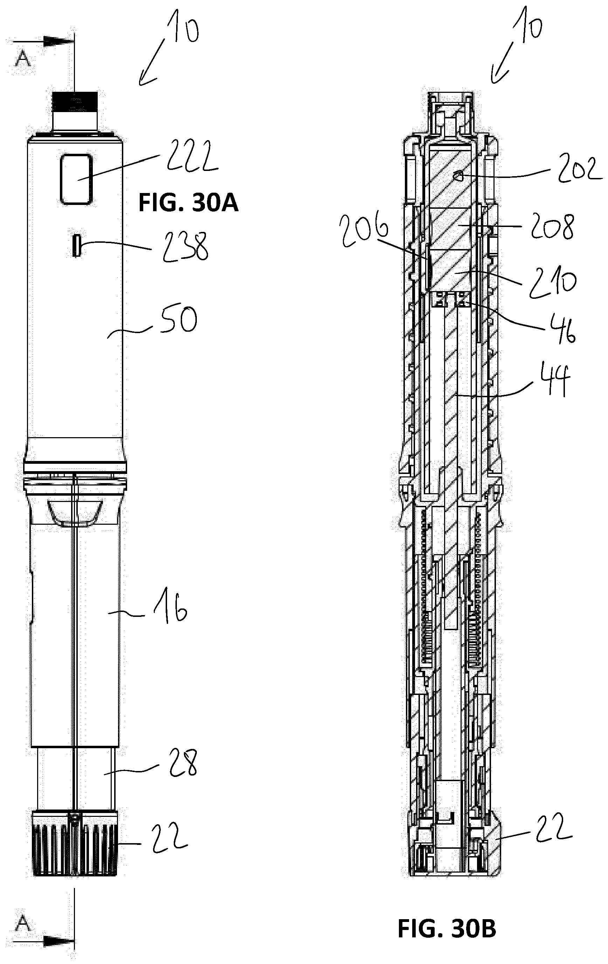

Afterwards, as can be seen when comparing Figs 29A and 30A, the dose setting knob 22 is rotated by the user the set a desired dose out of multiple possible settable doses. In this example, the dose setting knob 22 is rotated 180° to set the desired dose. While the dose setting knob 22 is rotated, the dose setting knob 22 makes a compulsory guided combined axial and rotational movement, namely a screw movement, in the distal direction.

Rotating the dose setting knob 22 causes rotation of the injection button 18, that is axially and rotationally connected to the dose setting knob 22 via the snap ring 20, the snap element 24, which is rotationally connected to the dose setting knob 22 via the teeth 108 intermeshing with the teeth 110, the driver 36, which is rotationally and axially coupled to the snap element 24, and the dose setting sleeve 34 which is rotationally and axially coupled to the driver 36. Rotation of the driver 36 causes the driver 36 to move axially in a distal direction due to the engagement of the outer thread 170 of the driver 36 and the inner thread 172 of the piston rod guide 42. The axial movement of the driver 36 causes the snap element 24 to move in a distal direction which pushes the injection button 18 and the dose setting knob 22 in the distal direction via the couplings means 102 of the snap element 24 interacting with the assembling means 98 of the injection button 18. This causes the dose setting knob 22 to perform a compulsory guided combined axial and rotational movement during dose setting.

Furthermore, rotating the dose setting knob 22 causes rotation of the injection button 18 that is rotationally coupled to the nut 38. Since the piston rod 44 is rotationally fixedly coupled to the piston rod guide 42 due to their corresponding out of round cross-sections 186, 188, the nut 38 moves in the distal direction when the dose setting knob 22 and therefore the nut 38 is rotated.

The amount of axial movement of the nut 38 relative to the piston rod 44 and the driver 36 relative to the piston guide 42 depends on the pitch of the respective thread. The outer thread 170 of the driver 36 has a greater pitch than the outer thread 190 of the piston rod 44 so that the driver 36 moves in the distal direction more than the nut 38. For example, the outer thread 170 of the driver 36 can have a pitch of 10.71 mm and the outer thread 190 of the piston rod 44 can have a pitch of 10.21 mm.

When the desired dose is set, the spiral torsion spring 40 applies a torque to the snap element 24 via the driver 36 to bring the dose definition element 116 in abutment with the respective dose stop 118a to 118d, namely with its side surface 122b. Due to the spring 40, the injection pen 10 is configured to rotationally self-align the snap element 24 and the dose selector 28 in different predefined rotational positions defining predefined doses.

If the user then pushes the injection button 18 on the distal end 12 of the injection pen 10, the dose setting knob 22 moves in the proximal direction relative to the snap element 24. This results in the coupling means 100 being bend while passing the circumferential ledge 102 causes a counterforce in the distal direction which has to be overcome by the user to start the injections process. The dose setting knob 22 moving in the proximal direction relative to the snap element 24 also results in the teeth 108 of the dose setting knob 22 disengaging with the teeth 110 of the snap element 24 and instead the teeth 108 of the dose setting knob 22 engaging with the teeth 114 of the connector 26. Since the connector 26 is rotationally coupled to the housing 32 via the dose selector 28, the dose setting knob 22 is rotationally fixed to the housing 32. Therefore, during dose delivery, the dose setting knob 22, the injection button 18, the dose selector 28, and the nut 38 do not rotate relative to the housing 32.

If the user further pushes injection button 18, the injection button 18 and the dose selector 28 move relative to the snap element 24 in the proximal direction. Thereby, the dose definition element 116 of the snap element 24 passes through the circumferentially extending rib 156 on the dose selector 28 through the respective cut-out 158a-158d corresponding to the set dose. At the same time, the hard stop 126 of the dose selector 28 moves in the axial direction relative to the hard stop 124 on the snap element 24 which allows the dose selector 28 and the snap element 24 to rotate relative to each other past the pre-set dose position towards the zero-dose position.

When the injection button 18 is pushed during dose delivery, the injection button 18 pushes the driver 36 via the snap element 24 in the proximal direction. The spring 40 supports the axial movement of the driver 36 by applying a torque to the driver 36 resulting in an axial movement of the driver 36 in the proximal direction due to the outer thread 170 of the driver 36. The driver pushes the nut 38 in the proximal direction which causes the piston rod 44 to move in the proximal direction. The movement of the piston rod 44 and the piston disc 46 in the proximal direction causes the drug to be injected into the patient. Since the injection pen 10 is made to inject relatively large amounts of drug, the pen 10 does not have a so-called gearing. In other words, the parts that are configured to rotate relative to the housing during dose delivery are connected to the housing 32. This means that the distance the piston disc 46 advances is essentially equal to the distance the injection button 18 is pushed in the proximal direction relative to the housing 32.

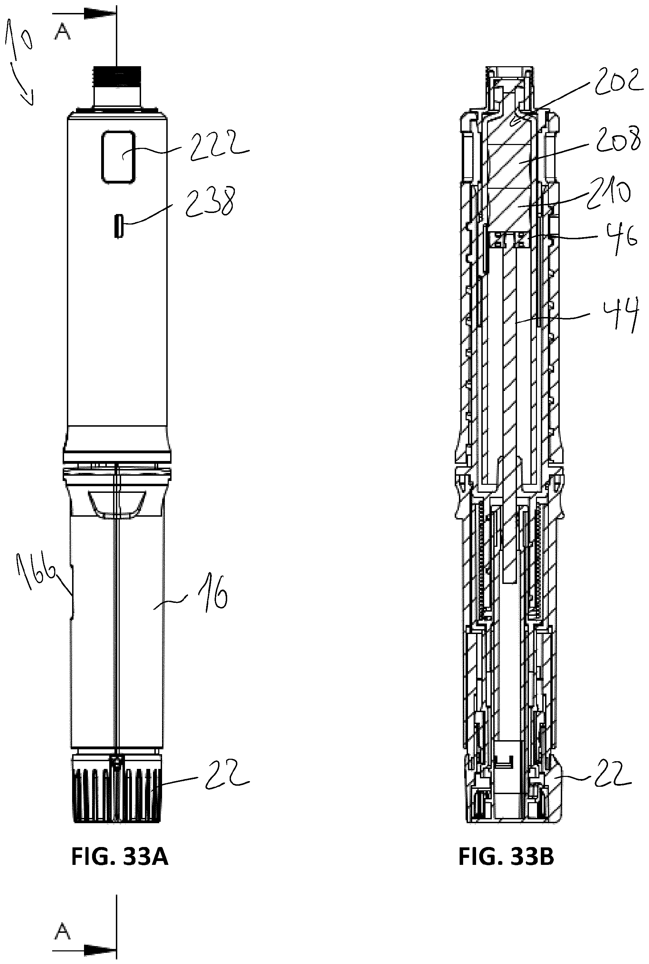

Since the driver rotates relative to the housing due to its outer thread 170, the dose setting sleeve 34 rotates during dose delivery. At the end of the dose delivery (cf. Fig. 33A and 33B) the dose setting sleeve 34 is in a rotational position in which a zero-dose label can be seen through the window 166 of the housing 32. The end of dose stop 174 (cf. Fig. 13B) of the driver 36 and the end of dose stop 176 (cf. Fig. 16C) of the piston rod guide 42 define an end of the movement of the injection button 18 in the proximal direction during dose delivery.

At the end of the dose delivery, the coupling means 100 on the injection button 18 passes the coupling means 102 of the snap element 24 when initiating the injection, which permanently rotationally couples the dose setting knob 22 and the injection button 18 to the housing 32. Thus, the injection pen 10 is rendered inoperable, as the user cannot rotate the dose setting knob 22 to set a new dose.

### List of reference signs

- 10: injection pen
- 12: distal end
- 14: proximal end
- 16: knob cover
- 16a: closed circumference
- 16b: closed face
- 18: injection button
- 18a: cylindrical portion
- 20: snap ring
- 22: dose setting knob
- 24: snap element
- 26: connector
- 28: dose selector
- 30: knob key
- 32: housing
- 32a: surface
- 34: dose setting sleeve
- 36: driver
- 38: nut
- 40: spring
- 42: piston rod guide
- 44: piston rod
- 46: piston disc
- 48: dual chamber cartridge
- 50: cartridge container
- 52: cartridge key
- 54: dose setting mechanism
- 56: drug reconstitution unit
- 58: wing
- 60: lug
- 62: window
- 64: elevation
- 66: abutment
- 66a: front surface
- 68: cut-out
- 69: chamfered portion
- 70: axial abutment elevation
- 72: clearance
- 74: linear recess
- 76: anti-rolling means
- 78: anti-rolling means
- 80: front surface
- 82: axial fixation means
- 84: rib
- 86: axial fixation means
- 88: undercut
- 90: rotation fixation means
- 92: rotation fixation means
- 94: rotation fixation means
- 96: rotation fixation means
- 98: assembling means
- 100: coupling means
- 100a: chamfered surface
- 101: cut-out
- 102: coupling means
- 104: rib
- 106: groove
- 108: teeth
- 110: teeth
- 112: coupling surface
- 114: teeth
- 116: engagement feature
- 118a: dose stop
- 118b: dose stop
- 118c: dose stop
- 118d: dose stop
- 120: elastically deformable section
- 121: cut-out
- 122a-d: side surface
- 123a-d: side surface
- 124: hard stop
- 126: hard stop
- 128: hard stop
- 130: opening
- 132: slot
- 134: rib
- 136: protrusion
- 136a: chamfered surface
- 138: rib
- 140: groove
- 142: axial fixation means
- 144: intake
- 146: axial fixation means
- 148: rotation fixation means
- 150: rotation fixation means
- 152: rotation fixation means
- 154: rotation fixation means
- 156: rib
- 158a: cut-out
- 158b: cut-out
- 158c: cut-out
- 158d: cut-out
- 160: proximal edge
- 162: distal edge
- 164: holding protrusion
- 166: window
- 168a: label
- 168b: label
- 168c: label
- 168d: label
- 168e: label
- 169a: out-of-round outer circumferential surface
- 169b: out-of-round inner circumferential surface
- 170: outer thread
- 171: fixing section
- 172: inner thread
- 173: face surface
- 174: end stop
- 176: end stop
- 177: attachment means
- 178: axial fixation means
- 179: attachment means
- 180: axial fixation means
- 182: rotation fixation means
- 184: rotation fixation means
- 186: opening
- 188: cross-section
- 190: outer thread
- 192: inner thread
- 194: pressing surface
- 196: front surface
- 198: coupling means
- 200: coupling means
- 202: first chamber
- 204: second chamber
- 206: bypass
- 208: first sealing element
- 210: second sealing element
- 212: axial fixation means
- 214: axial fixation means
- 216: rotation fixations means
- 218: rotation fixations means
- 220: window
- 221: cut-out
- 222: window
- 223: slot
- 224: thread
- 226: end surface
- 228: surface
- 230: first thread
- 232: second thread
- 234: snap element
- 236: opening
- 238: opening
- 240: opening
- 242: displacement section
- 244: radial end stop

- L1: length
- L2: length
- W1: width
- W2: width

## Claims

1. A dose setting mechanism (54) configured to set a desired dose for an injection device (10), the dose setting mechanism (54) comprising a housing (32), a dose setting knob (18, 22) and a dose setting device (24),
wherein said dose setting knob (22) is configured to set the desired dose by a compulsory guided combined axial and rotational movement of the dose setting knob (22) relative to the housing (32), wherein said dose setting mechanism (54) comprises one or more coupling means (100),
and wherein, on drug delivery, said dose setting knob (18, 22) is moved axially relative to the dose setting device (24) and/or the housing (32) and said one or more coupling means (100) are configured to engage at least one of the dose setting device (24) and the housing (32) to thereby permanently axially lock the dose setting knob (18, 22) to the respective one of the dose setting device (24) and the housing (32).

2. The dose setting mechanism (54) according to claim 1, wherein the dose setting knob (18, 22) is non-rotatable relative to the dose setting device (24) and/or the housing (32) when the dose setting knob (18, 22) is axially locked to the respective one of the dose setting device (24) and the housing (32).

3. The dose setting mechanism (54) according to claim 1 or 2,
wherein said one or more coupling means (100) are configured to cooperate between the dose setting device (24) and the dose setting knob (18, 22).

4. The dose setting mechanism (54) according to any one of the preceding claims,
wherein said dose setting knob (18, 22) comprises said one or more coupling means (100) configured to cooperate with the respective one of the dose setting device (24) and the housing (32).

5. The dose setting mechanism (54) according to any one of the preceding claims,
wherein the one or more coupling means (100) are arranged on an outer circumferential surface of the dose setting knob (18, 22) and corresponding coupling means (102), e.g. a circumferentially extending ledge, are arranged on an inner circumferential surface of the dose setting device (24).

6. The dose setting mechanism (54) according to any one of the preceding claims,
wherein the one or more coupling means (100) comprise one or more snap elements, in particular one or more elastically deformable tongues,
preferably wherein the one or more snap elements have a chamfered surface that is configured to engage with a protrusion (102), in particular a circumferentially extending ledge, to elastically deform the snap element.

7. The dose setting mechanism (54) according to any one of the preceding claims,
wherein a cut-out (101) is provided next to at least one of the one or more coupling means (100), preferably
wherein the cut-out (101) is provided sectionally around the at least one of the one or more coupling means (100).

8. The dose setting mechanism (54) according to any one of the preceding claims,
wherein the dose setting device (24) comprises one or more projections (116) for setting said desired dose and cooperating with a dose selector (28)
and/or
wherein the dose setting knob (18, 22) is coupled to a dose selector (28) in a rotationally fixed manner on drug delivery and/or wherein the dose setting knob (18, 22) rotates relative to the housing (32) while the dose selector (28) does not rotate relative to the housing (32) on dose setting.

9. The dose setting mechanism (54) according to any one of the preceding claims,
wherein the dose setting knob (18, 22) forms an end face for manually pushing the dose setting knob (18, 22) in an axial direction relative to the dose setting device (24) and/or the housing (32) on drug delivery,
and/or
wherein the dose setting knob (18, 22) and the dose setting device (24) are axially moveable relative to one another on dose setting by rotating the dose setting knob (18, 22) relative to the housing (32),
and/or
wherein the dose setting device (24) and the dose setting knob (18, 22) are rotationally fixed to each other during dose setting and/or wherein the dose setting device (24) and the dose setting knob (18, 22) rotate relative to each other on drug delivery.

10. The dose setting mechanism (54) according to any one of the preceding claims,
wherein the dose setting device (24) rotates relative to the housing (32) on drug delivery and/or the dose setting knob (18, 22) does not rotate relative to the housing (32) on drug delivery and/or
wherein the dose setting knob (18, 22) and the dose setting device (24) are axially moveable relative to the housing (32) on dose setting,
and/or
wherein the dose setting knob (18, 22) and the dose setting device (24) are configured to not move relative to one another on dose setting.

11. The dose setting mechanism (54) according to any one of the preceding claims,
wherein the dose setting knob (18, 22) comprises an injection button (18) and said one or more coupling means (100) are configured to permanently axially lock said injection button (18) to the respective one of the dose setting device (24) and the housing (32),
and/or
wherein at least a part of the dose setting knob (18, 22) is axially moved on dose dispensing, and/or
wherein the dose setting knob (18, 22) comprises an injection button (18) and said injection button (18) is axially moved on dose dispensing,
and/or
wherein, on drug delivery, said dose setting knob (18, 22) is moved axially relative to said dose setting device (24) and said one or more coupling means (100) are configured to engage the dose setting device (24) to thereby permanently axially lock the dose setting knob (18, 22) to the dose setting device (24) due to the relative movement of the dose setting knob (18, 22) and the dose setting device (24).

12. A medicament delivery device (10), in particular an injection device, comprising a dose setting mechanism (54) according to any one of the preceding claims,
and/or a single use and single dose pen with one or more doses being selectable for the single use with a dose setting mechanism (54) according to any one of the preceding claims.

13. Set comprising a medicament delivery device or a pen according to claim 12, and a separate element (30),
wherein the separate element (30) is provided to prevent a premature moving of the dose setting knob (22) and/or the injection button (18), relative to the housing (32) before the separate element (30) is removed, preferably wherein a holding element, e.g. a knob cover (16), is provided that has to be removed before the separate element (30) can be removed.

14. Method of locking a medicament delivery device (10), in particular an injection pen (10), following first use of the injection pen (10), the method comprising the steps of:
setting a dose by a compulsory guided combined axial and rotational movement of the dose setting knob (18, 22) relative to the housing (32), and
moving a dose setting knob (18, 22) of a dose setting mechanism (54) axially relative to a dose setting device (24) and/or a housing (32), thereby activating one or more coupling means (100) to permanently axially lock the dose setting knob (18, 22).

15. Method of locking a medicament delivery device (10), in particular an injection pen (10), according to claim 14,
the method comprising the steps of:
moving said dose setting knob (18, 22) of the dose setting mechanism (54) axially relative to the dose setting device (24) and/or the housing (32), thereby moving one or more coupling means (100) of the dose setting knob (18, 22) into engagement with the dose setting device (24) or the housing (32) and axially locking the dose setting knob (18, 22) to the dose setting device (24) and/or the housing (32),
and/or
wherein at least a part of the dose setting knob (18, 22) is axially moved on dose dispensing,
and/or
wherein the dose setting knob (18, 22) comprises an injection button (18) and wherein the injection button (18) is axially moved on dose dispensing,
and/or
wherein the moving of the dose setting knob (18, 22) of the dose setting mechanism (54) axially relative to the dose setting device (24) and/or the housing (32) is done by manually pushing the dose setting knob (18, 22) in an axial direction,
and/or
wherein the dose setting knob (18, 22) is axially moved backwards relative to the housing (32) to set a desired dose for a medicament delivery device, in particular for an injection device (10), prior to moving of the dose setting knob (18, 22) of the dose setting mechanism (54) axially forward relative to the dose setting device (24) and/or the housing (32),
and/or
wherein the dose setting knob (18, 22) is axially moved backwards relative to the housing (32) by rotating the dose setting knob (18, 22) relative to the housing.
